(19) Europäisches Patentamt
European Patent Office
Office européen des brevets

(11) Veröffentlichungsnummer: **0 047 817**
**A2**

# (12) EUROPÄISCHE PATENTANMELDUNG

(21) Anmeldenummer: **81104980.8**

(22) Anmeldetag: **26.06.81**

(51) Int. Cl.$^3$: **C 07 C 121/60**
C 09 K 3/34, C 07 C 43/20
C 07 C 43/235, C 07 C 43/18
C 07 C 69/753, C 07 C 103/19
C 07 C 103/22, C 07 C 13/48
C 07 C 13/50

(30) Priorität: **14.08.80 CH 6130/80**
**27.05.81 CH 3482/81**

(43) Veröffentlichungstag der Anmeldung:
**24.03.82 Patentblatt 82/12**

(84) Benannte Vertragsstaaten:
**CH DE FR IT LI**

(71) Anmelder: **F. HOFFMANN-LA ROCHE & CO.**
**Aktiengesellschaft**

**CH-4002 Basel(CH)**

(72) Erfinder: **Boller, Arthur, Dr.**
**Benkenstrasse 65**
**CH-4102 Binningen(CH)**

(72) Erfinder: **Schadt, Martin, Dr.**
**Liestalerstrasse 77**
**CH-4411 Seltisberg(CH)**

(72) Erfinder: **Villiger, Alois, Dr.**
**Im Ettingerhof 5**
**CH-4055 Basel(CH)**

(74) Vertreter: **Cottong, Norbert A. et al,**
**Grenzacherstrasse 124 Postfach 3255**
**CH-4002 Basel(CH)**

## (54) Hydrierte Naphthaline, deren Herstellung und Verwendung sowie derartige Naphthaline enthaltende Gemische.

(57) Verbindungen der allgemeinen Formel

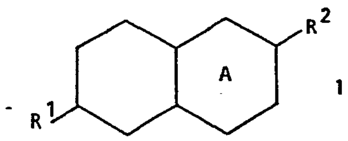

I

worin Ring A gesättigt oder aromatisch ist und ein gegebenenfalls vorhandener gesättigter Ring A mit dem zweiten Ring trans-verknüpft ist; $R^1$ eine geradkettige Alkyl- oder Alkoxygruppe mit 1 bis 11 Kohlenstoffatomen bezeichnet; $R^2$ Cyano, eine geradkettige Alkylgruppe mit 1 bis 11 Kohlenstoffatomen, eine Estergruppe der allgemeinen Formel

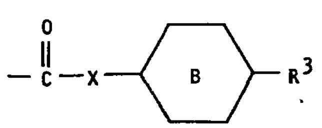

II

oder, sofern Ring A gesättigt ist, zusätzlich eine geradkettige Alkoxygruppe mit 1 bis 11 Kohlenstoffatomen bedeutet; in der Estergruppe der Formel II Ring B entweder aromatisch ist und X Sauerstoff oder Schwefel und $R^3$ Cyano oder eine geradkettige Alkyl- oder Alkoxygruppe mit 1 bis 10 Kohlenstoffatomen bezeichnet, oder Ring B einen trans-1,4-disubstituierten Cyclohexanring und X Sauerstoff und $R^3$ Cyano oder eine geradkettige Alkylgruppe mit 1 bis 10 Kohlenstoffatomen bedeutet; und die Gesamtzahl der Kohlenstoffatome in den Alkyl- und/oder Alkoxygruppen höchstens 12 beträgt,
deren Herstellung und Verwendung in elektro-optischen Vorrichtungen, flüssigkristalline Mischungen sowie neue Zwischenprodukte werden beschrieben.

Die neuen Verbindungen der Formel I sind besonders wertvoll als Komponenten in Flüssigkristallmischungen.

EP 0 047 817 A2

F.Hoffmann-La Roche & Co. Aktiengesellschaft,Basel,Schweiz

RAN 6220/020

# Hydrierte Naphthaline, deren Herstellung und Verwendung sowie derartige Naphthaline enthaltende Gemische

Die vorliegende Erfindung betrifft neue hydrierte Naphthaline der allgemeinen Formel

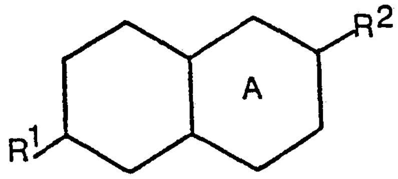

I

worin Ring A gesättigt oder aromatisch ist und ein gegebenenfalls vorhandener gesättigter Ring A mit dem zweiten Ring trans-verknüpft ist; $R^1$ eine geradkettige Alkyl- oder Alkoxygruppe mit 1 bis 11 Kohlenstoffatomen bezeichnet; $R^2$ Cyano, eine geradkettige Alkylgruppe mit 1 bis 11 Kohlenstoffatomen, eine Estergruppe der allgemeinen Formel

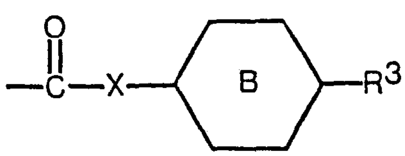

II

Zim/ 16.6.81

oder, sofern Ring A gesättigt ist, zusätzlich eine geradkettige Alkoxygruppe mit 1 bis 11 Kohlenstoffatomen bedeutet; in der Estergruppe der Formel II Ring B entweder aromatisch ist und X Sauerstoff oder Schwefel und $R^3$ Cyano oder eine geradkettige Alkyl- oder Alkoxygruppe mit 1 bis 10 Kohlenstoffatomen bezeichnet, oder Ring B einen trans-1,4-disubstituierten Cyclohexanring und X Sauerstoff und $R^3$ Cyano oder eine geradkettige Alkylgruppe mit 1 bis 10 Kohlenstoffatomen bedeutet; und die Gesamtzahl der Kohlenstoffatome in den Alkyl- und/oder Alkoxygruppen höchstens 12 beträgt.

Die Erfindung betrifft ferner die Herstellung der Verbindungen der obigen Formel I, flüssigkristalline Mischungen, welche diese Verbindungen enthalten, sowie deren Verwendung in elektro-optischen Vorrichtungen.

Der Ausdruck "geradkettige Alkylgruppe" bedeutet im Rahmen der vorliegenden Erfindung je nach Zahl der angegebenen Kohlenstoffatome Methyl, Aethyl, Propyl, Butyl, Pentyl, Hexyl, Heptyl, Octyl, Nonyl, Decyl und Undecyl und der Ausdruck "geradkettige Alkoxygruppe" entsprechend Methoxy, Aethoxy, Propyloxy, Butyloxy, Pentyloxy, Hexyloxy, Heptyloxy, Octyloxy, Nonyloxy, Decyloxy und Undecyloxy.

Die erfindungsgemässen Verbindungen sind besonders wertvoll als Komponenten in flüssigkristallinen Mischungen. Die Verbindungen der Formel I, worin $R^2$ eine Estergruppe der Formel II bedeutet, sind zum grössten Teil selbst flüssigkristallin. Die übrigen Verbindungen der Formel I, d.h. diejenigen worin $R^2$ Cyano oder eine geradkettige Alkyl- oder Alkoxygruppe bedeutet, sind vor allem als Dotierungsmittel in Flüssigkristallmischungen geeignet.

Diejenigen Verbindungen der Formel I, welche eine Cyanogruppe besitzen, weisen eine hohe positive Anisotro-

pie der Dielektrizitätskonstanten auf ($\varepsilon_{\parallel} > \varepsilon_{\perp}$, wobei $\varepsilon_{\parallel}$ die Dielektrizitätskonstante entlang der Moleküllängsachse und $\varepsilon_{\perp}$ die Dielektrizitätskonstante senkrecht dazu bedeuten). Demgegenüber besitzen die Verbindungen der Formel I, worin $R^2$ bzw. $R^3$ eine Alkylgruppe bedeutet, nur eine kleine Anisotropie der Dielektrizitätskonstanten.

In einem elektrischen Feld orientieren sich flüssigkristalline Verbindungen und Mischungen mit positiver Anisotropie der Dielektrizitätskonstanten mit der Richtung ihrer grössten Dielektrizitätskonstanten parallel zur Feldrichtung. Dieser Effekt wird u.a. in der von J.H. Heilmeier und L.A. Zanoni [Applied Physics Letters <u>13</u>, 91 (1968)] beschriebenen Wechselwirkung zwischen eingelagerten Molekülen und den flüssigkristallinen Molekülen (Guest-Host interaction) ausgenützt. Eine weitere interessante Anwendung der dielektrischen Feldorientierung liegt in der von M. Schadt und W. Helfrich [Applied Physics Letters <u>18</u>, (1971)] gefundenen Drehzelle sowie bei der in Molecular Crystals and Liquid Crystals <u>17</u>, 355 (1972) beschriebenen Kerrzelle vor.

Bei dieser elektro-optischen Drehzelle handelt es sich im wesentlichen um einen Kondensator mit lichtdurchlässigen Elektroden, dessen Dielektrikum von einem nematischen Kristall mit $\varepsilon_{\parallel} > \varepsilon_{\perp}$ gebildet wird. Die Moleküllängsachsen des flüssigen Kristalls sind im feldfreien Zustand schraubenförmig zwischen den Kondensatorplatten angeordnet, wobei die Schraubenstruktur durch die vorgegebene Wandorientierung der Moleküle bestimmt ist. Nach dem Anlegen einer elektrischen Spannung an die Kondensatorplatten stellen sich die Moleküle mit ihren Längsachsen in Feldrichtung (d.h. senkrecht zur Plattenoberfläche ein, wodurch linear polarisiertes Licht im Dielektrikum nicht mehr gedreht wird (der flüssige Kristall wird senkrecht zur Oberfläche der Platten einachsig). Dieser Effekt ist reversibel und kann dazu verwendet werden, die optische Transparenz des Kondensators elektrisch zu steuern.

Da die erfindungsgemässen Verbindungen je nach Art der Substituenten unterschiedliche Anisotropie der Dielektrizitätskonstanten aufweisen, können sie dazu verwendet werden, die Schwellenspannung von Mischungen der verwendeten elektro-optischen Zelle anzupassen.

Es wurde ferner gefunden, dass die Verbindungen der Formel I, worin $R^2$ eine Estergruppe der Formel II darstellt, einen besonders grossen nematischen Mesophasenbereich aufweisen, insbesondere im Vergleich zu bekannten Estern mit ähnlich niedrigen Schmelzpunkten. Zudem besitzen sie eine gute chemische Stabilität, niedere Viskosität, leichte Orientierbarkeit, geringe smektische Tendenzen und sie ergeben in Anzeigevorrichtungen einen hohen Kontrast.

Die Verbindungen der Formel I, worin $R^2$ Cyano, Alkyl oder Alkoxy bedeutet, besitzen eine ausgezeichnete chemische Stabilität und niedere Viskosität. Sie sind daher besonders geeignet, die Viskosität und somit auch die elektro-optische Ansprechzeit von flüssigkristallinen Mischungen zu verbessern. Die hohen Siedepunkte dieser Verbindungen (im Vergleich zu bisher gebräuchlichen Dotierungsmitteln) erleichtern zudem die exakte Herstellung von Mischungen in vorgegebenen Mengenverhältnissen, da hierbei die Verdunstung sehr gering gehalten werden kann.

Die erfindungsgemässen Verbindungen sind farblos und weisen eine hohe UV-Stabilität auf, da sie bei kurzen Wellenlängen und zudem mit kleinen Extinktionen absorbieren. Die optische Anisotropie ist je nach Molekülstruktur verschieden; im allgemeinen ist sie umso grösser, je grösser der ungesättigte Molekülteil ist. Durch geeignete Wahl der erfindungsgemässen Verbindungen kann deshalb die optische Anisotropie von Mischungen variiert, d.h. deren Kontrast in einer gegebenen Zelle optimiert werden.

Die erfindungsgemässen Verbindungen sind ferner gut

mischbar mit allen bekannten Flüssigkristallen.

Die unter die allgemeine Formel I fallenden, erfindungsgemässen Verbindungen sind entweder trans-Decaline der allgemeinen Formel

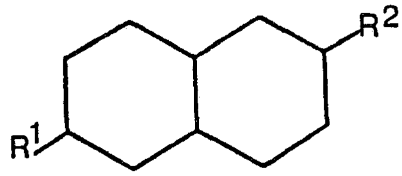

IA

oder Tetraline (1,2,3,4-Tetrahydronaphthaline) der allgemeinen Formel

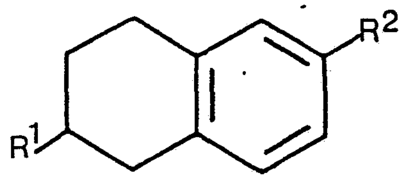

IB

in denen die Reste $R^1$ und $R^2$ die obige Bedeutung haben.

Die erfindungsgemässen Verbindungen weisen mindestens ein chirales Zentrum auf und können daher in optisch aktiver oder racemischer Form vorliegen.

Die im folgenden genannten Verbindungen der Formel I liegen jedoch – sofern nicht ausdrücklich etwas anderes angegeben ist – im allgemeinen als Racemate vor, und zwar die Verbindungen der Formel IA als Racemat bestehend aus den Verbindungen der Formeln

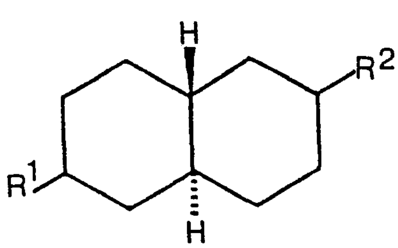

und

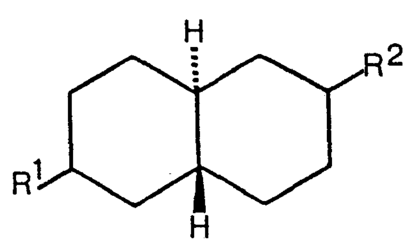

und die Verbindungen der Formel IB als Racemat bestehend aus den Verbindungen der Formeln

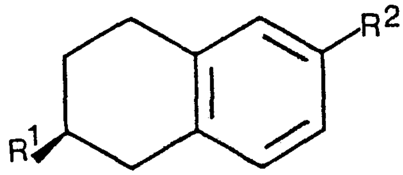

und

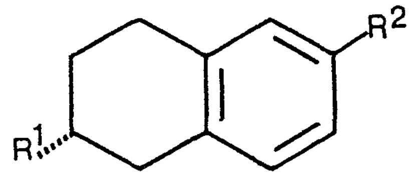

Das Symbol (▶) bedeutet, dass die entsprechende Bindung zum Substituenten hin nach oben (über die Zeichenebene; β-Stellung) gerichtet ist, und das Symbol (ıııııı) dass sie nach unten (unter die Zeichenebene; α-Stellung) gerichtet ist.

Im Rahmen der vorliegenden Erfindung wurde auch für die übrigen Decaline und Tetraline eine den Formeln I, IA und IB analoge Darstellung verwendet.

Aufgrund des $^1$H-NMR-Spektrums einiger erfindungsgemäss hergestellter Verbindungen wird angenommen, dass der Rest $R^2$ in den Verbindungen der Formel IA in äquatorialer Position steht. Die Stellung des Substituenten $R^1$ konnte nicht abgeklärt werden. Aufgrund der ausgeprägten flüssigkristallinen Eigenschaften der Decalinester wird jedoch vermutet, dass auch der Substituent $R^1$ in äquatorialer Position steht. Sofern diese Vermutung zutrifft, wären die Verbindungen der Formel IA Racemate bestehend aus den Verbindungen der Formeln

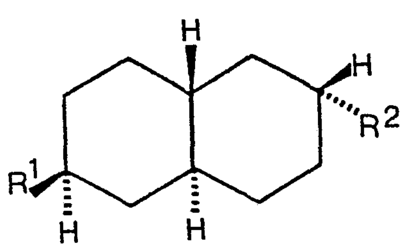

und

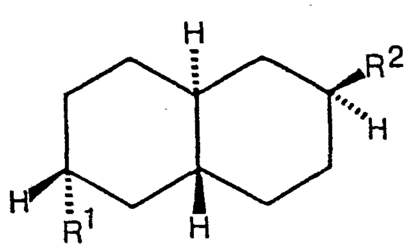

worin $R^1$ und $R^2$ die obige Bedeutung haben. Eine Ausnahme

bilden jedoch die Verbindungen der Formel IA, worin $R^1$ und $R^2$ die gleiche Bedeutung haben, da in diesem Fall die beiden obigen Formeln identisch sind und einer optisch inaktiven Form entsprechen.

Die obige Formel I umfasst vorzugsweise die racemischen Verbindungen.

Weiterhin sind von den Verbindungen der obigen Formeln IA und IB diejenigen der Formel IA bevorzugt. Von den Verbindungen der Formel I sind ferner diejenigen bevorzugt, worin $R^2$ eine Estergruppe der Formel II bedeutet. Bevorzugte Estergruppen der Formel II sind diejenigen, worin Ring B aromatisch ist, und ferner diejenigen, worin X Sauerstoff bedeutet und bevorzugte Reste $R^3$ sind die Cyanogruppe und die Alkylgruppen, insbesondere die Cyanogruppe. Besonders bevorzugte Flüssigkristallkomponenten sind somit die trans-Decalin-2-carbonsäure-phenylester und insbesondere die trans-Decalin-2-carbonsäure-p-cyanophenylester. Bevorzugte Dotierungsmittel sind diejenigen Verbindungen der Formel I, worin $R^2$ die Cyano- oder eine Alkylgruppe, insbesondere die Cyanogruppe bedeutet. Bevorzugte Reste $R^1$ in den Verbindungen der Formel I sind die Alkylgruppen. Bevorzugte Alkyl- und Alkoxygruppen $R^1$ bzw. $R^2$ sind diejenigen mit 1 bis 8 Kohlenstoffatomen, insbesondere diejenigen mit 3 bis 7 Kohlenstoffatomen. Von den Alkyl- und Alkoxygruppen $R^3$ in der Estergruppe der Formel II sind diejenigen mit 1 bis 7 Kohlenstoffatomen bevorzugt und diejenigen mit 1 bis 5 Kohlenstoffatomen besonders bevorzugt.

Als Beispiele bevorzugter Verbindungen der Formel I können folgende genannt werden:

6-Methyl-trans-decalin-2-carbonsäure-p-cyanophenylester,

6-Aethyl-trans-decalin-2-carbonsäure-p-cyanophenylester,

6-Propyl-trans-decalin-2-carbonsäure-p-cyanophenylester,
6-Butyl-trans-decalin-2-carbonsäure-p-cyanophenylester,
6-Pentyl-trans-decalin-2-carbonsäure-p-cyanophenylester,
6-Hexyl-trans-decalin-2-carbonsäure-p-cyanophenylester,
6-Heptyl-trans-decalin-2-carbonsäure-p-cyanophenylester,
6-Octyl-trans-decalin-2-carbonsäure-p-cyanophenylester,
6-Methyl-trans-decalin-2-carbonsäure-p-butylphenylester,
6-Aethyl-trans-decalin-2-carbonsäure-p-äthylphenyl ester,
6-Propyl-trans-decalin-2-carbonsäure-p-methylphenylester,
6-Butyl-trans-decalin-2-carbonsäure-p-methylphenylester,
6-Pentyl-trans-decalin-2-carbonsäure-p-methylphenylester,
6-Hexyl-trans-decalin-2-carbonsäure-p-methylphenylester,
6-Heptyl-trans-decalin-2-carbonsäure-p-methylphenylester,
6-Propyl-trans-decalin-2-carbonsäure-p-äthylphenylester,
6-Butyl-trans-decalin-2-carbonsäure-p-äthylphenylester,
6-Pentyl-trans-decalin-2-carbonsäure-p-äthylphenylester,
6-Hexyl-trans-decalin-2-carbonsäure-p-äthylphenylester,
6-Heptyl-trans-decalin-2-carbonsäure-p-äthylphenylester,

6-Propyl-trans-decalin-2-carbonsäure-p-propylphenylester,

6-Butyl-trans-decalin-2-carbonsäure-p-propylphenylester,

6-Pentyl-trans-decalin-2-carbonsäure-p-propylphenylester,

6-Hexyl-trans-decalin-2-carbonsäure-p-propylphenylester,

6-Heptyl-trans-decalin-2-carbonsäure-p-propylphenylester,

6-Propyl-trans-decalin-2-carbonsäure-p-butylphenylester,

6-Butyl-trans-decalin-2-carbonsäure-p-butylphenylester,

6-Pentyl-trans-decalin-2-carbonsäure-p-butylphenylester,

6-Hexyl-trans-decalin-2-carbonsäure-p-butylphenylester,

6-Heptyl-trans-decalin-2-carbonsäure-p-butylphenylester,

6-Propyl-trans-decalin-2-carbonsäure-p-pentylphenylester,

6-Butyl-trans-decalin-2-carbonsäure-p-pentylphenylester,

6-Pentyl-trans-decalin-2-carbonsäure-p-pentylphenylester,

6-Hexyl-trans-decalin-2-carbonsäure-p-pentylphenylester,

6-Heptyl-trans-decalin-2-carbonsäure-p-pentylphenylester,

6-Propyl-trans-decalin-2-carbonsäure-p-methoxyphenylester,

6-Pentyl-trans-decalin-2-carbonsäure-p-methoxyphenylester,

6-Heptyl-trans-decalin-2-carbonsäure-p-methoxyphenylester,

6-Propyl-trans-decalin-2-carbonsäure-p-äthoxyphenyl-ester,

6-Pentyl-trans-decalin-2-carbonsäure-p-äthoxyphenyl-ester,

6-Heptyl-trans-decalin-2-carbonsäure-p-äthoxyphenyl-ester,

6-Propyl-trans-decalin-2-carbonsäure-p-propyloxy-phenylester,

6-Pentyl-trans-decalin-2-carbonsäure-p-propyloxy-phenylester,

6-Heptyl-trans-decalin-2-carbonsäure-p-propyloxy-phenylester,

6-Propyl-trans-decalin-2-carbonsäure-p-pentyloxy-phenylester,

6-Pentyl-trans-decalin-2-carbonsäure-p-pentyloxy-phenylester,

6-Methoxy-trans-decalin-2-carbonsäure-p-cyanophenyl-ester,

6-Aethoxy-trans-decalin-2-carbonsäure-p-cyanophenyl-ester,

6-Propyloxy-trans-decalin-2-carbonsäure-p-cyano-phenylester,

6-Butyloxy-trans-decalin-2-carbonsäure-p-cyano-phenylester,

6-Pentyloxy-trans-decalin-2-carbonsäure-p-cyano-phenylester,

6-Hexyloxy-trans-decalin-2-carbonsäure-p-cyano-phenylester,

6-Heptyloxy-trans-decalin-2-carbonsäure-p-cyano-phenylester,

6-Octyloxy-trans-decalin-2-carbonsäure-p-cyano-phenylester,

6-Propyloxy-trans-decalin-2-carbonsäure-p-methyl-phenylester,

6-Pentyloxy-trans-decalin-2-carbonsäure-p-methyl-phenylester,

6-Heptyloxy-trans-decalin-2-carbonsäure-p-methyl-phenylester,

6-Propyloxy-trans-decalin-2-carbonsäure-p-propylphenylester,

6-Pentyloxy-trans-decalin-2-carbonsäure-p-propylphenylester,

6-Heptyloxy-trans-decalin-2-carbonsäure-p-propylphenylester,

6-Propyloxy-trans-decalin-2-carbonsäure-p-pentylphenylester,

6-Pentyloxy-trans-decalin-2-carbonsäure-p-pentylphenylester,

6-Heptyloxy-trans-decalin-2-carbonsäure-p-pentylphenylester,

6-Propyl-trans-decalin-2-carbonsäure-p-cyanophenylthioester,

6-Butyl-trans-decalin-2-carbonsäure-p-cyanophenylthioester,

6-Pentyl-trans-decalin-2-carbonsäure-p-cyanophenylthioester,

6-Hexyl-trans-decalin-2-carbonsäure-p-cyanophenylthioester,

6-Heptyl-trans-decalin-2-carbonsäure-p-cyanophenylthioester,

6-Propyl-trans-decalin-2-carbonsäure-trans-4-cyano-1-cyclohexylester,

6-Butyl-trans-decalin-2-carbonsäure-trans-4-cyano-1-cyclohexylester,

6-Pentyl-trans-decalin-2-carbonsäure-trans-4-cyano-1-cyclohexylester,

6-Hexyl-trans-decalin-2-carbonsäure-trans-4-cyano-1-cyclohexylester,

6-Heptyl-trans-decalin-2-carbonsäure-trans-4-cyano-1-cyclohexylester,

6-Propyl-trans-decalin-2-carbonsäure-trans-4-methyl-1-cyclohexylester,

6-Pentyl-trans-decalin-2-carbonsäure-trans-4-methyl-1-cyclohexylester,

6-Heptyl-trans-decalin-2-carbonsäure-trans-4-methyl-1-cyclohexylester,

6-Propyl-trans-decalin-2-carbonsäure-trans-4-propyl-1-cyclohexylester,
6-Pentyl-trans-decalin-2-carbonsäure-trans-4-propyl-1-cyclohexylester,
6-Heptyl-trans-decalin-2-carbonsäure-trans-4-propyl-1-cyclohexylester,
6-Propyl-trans-decalin-2-carbonsäure-trans-4-pentyl-1-cyclohexylester,
6-Butyl-trans-decalin-2-carbonsäure-trans-4-pentyl-1-cyclohexylester,
6-Pentyl-trans-decalin-2-carbonsäure-trans-4-pentyl-1-cyclohexylester,
6-Heptyl-trans-decalin-2-carbonsäure-trans-4-pentyl-1-cyclohexylester,
6-Aethyl-trans-decalin-2-carbonitril,
6-Propyl-trans-decalin-2-carbonitril,
6-Pentyl-trans-decalin-2-carbonitril,
6-Heptyl-trans-decalin-2-carbonitril,
6-Pentyloxy-trans-decalin-2-carbonitril,
2-Aethyl-6-propyl-trans-decalin,
2-Aethyl-6-pentyl-trans-decalin,
2-Aethyl-6-heptyl-trans-decalin,
2,6-Dipropyl-trans-decalin,
2-Butyl-6-propyl-trans-decalin,
2-Pentyl-6-propyl-trans-decalin,
2-Pentyl-6-butyl-trans-decalin,
2,6-Dipentyl-trans-decalin,
2-Hexyl-6-propyl-trans-decalin,
2-Hexyl-6-pentyl-trans-decalin,
2-Heptyl-6-propyl-trans-decalin,
2-Heptyl-6-butyl-trans-decalin,
2-Heptyl-6-pentyl-trans-decalin,
2-Propyloxy-6-propyl-trans-decalin,
2-Propyloxy-6-pentyl-trans-decalin,
2-Propyloxy-6-heptyl-trans-decalin,
2-Pentyloxy-6-propyl-trans-decalin,
2-Pentyloxy-6-pentyl-trans-decalin,
2-Pentyloxy-6-heptyl-trans-decalin,

2-Heptyloxy-6-propyl-trans-decalin,

p-Cyanophenyl-2-methyl-1,2,3,4-tetrahydro-6-naphthoat,

p-Cyanophenyl-2-äthyl-1,2,3,4-tetrahydro-6-naphthoat,

p-Cyanophenyl-2-propyl,1,2,3,4-tetrahydro-6-naphthoat,

p-Cyanophenyl-2-butyl-1,2,3,4-tetrahydro-6-naphthoat,

p-Cyanophenyl-2-pentyl-1,2,3,4-tetrahydro-6-naphthoat,

p-Cyanophenyl-2-hexyl-1,2,3,4-tetrahydro-6-naphthoat,

p-Cyanophenyl-2-heptyl-1,2,3,4-tetrahydro-6-naphthoat,

p-Cyanophenyl-2-octyl-1,2,3,4-tetrahydro-6-naphthoat,

p-Methylphenyl-2-propyl-1,2,3,4-tetrahydro-6-naphthoat,

p-Methylphenyl-2-pentyl-1,2,3,4-tetrahydro-6-naphthoat,

p-Methylphenyl-2-heptyl-1,2,3,4-tetrahydro-6-naphthoat,

p-Propylphenyl-2-propyl-1,2,3,4-tetrahydro-6-naphthoat,

p-Propylphenyl-2-pentyl-1,2,3,4-tetrahydro-6-naphthoat,

p-Propylphenyl-2-heptyl-1,2,3,4-tetrahydro-6-naphthoat,

p-Butylphenyl-2-pentyl-1,2,3,4-tetrahydro-6-naphthoat,

p-Pentylphenyl-2-propyl-1,2,3,4-tetrahydro-6-naphthoat,

p-Pentylphenyl-2-pentyl-1,2,3,4-tetrahydro-6-naphthoat,

p-Pentylphenyl-2-heptyl-1,2,3,4-tetrahydro-6-naphthoat,

p-Cyanophenyl-2-propyloxy-1,2,3,4-tetrahydro-6-naphthoat,

p-Cyanophenyl-2-pentyloxy-1,2,3,4-tetrahydro-6-naphthoat,

p-Cyanophenyl-2-heptyloxy-1,2,3,4-tetrahydro-6-naph-

thoat,

p-Methylphenyl-2-pentyloxy-1,2,3,4-tetrahydro-6-naphthoat,

p-Propylphenyl-2-pentyloxy-1,2,3,4-tetrahydro-6-naphthoat,

p-Pentylphenyl-2-pentyloxy-1,2,3,4-tetrahydro-6-naphthoat,

p-Methoxyphenyl-2-pentyl-1,2,3,4-tetrahydro-6-naphthoat,

p-Aethoxyphenyl-2-pentyl-1,2,3,4-tetrahydro-6-naphthoat,

p-Propyloxyphenyl-2-pentyl-1,2,3,4-tetrahydro-6-naphthoat,

p-Aethoxyphenyl-2-heptyl-1,2,3,4-tetrahydro-6-naphthoat,

S-(p-Cyanophenyl)-2-propyl-1,2,3,4-tetrahydro-thio-6-naphthoat,

S-(p-Cyanophenyl)-2-butyl-1,2,3,4-tetrahydro-thio-6-naphthoat,

S-(p-Cyanophenyl)-2-pentyl-1,2,3,4-tetrahydro-thio-6-naphthoat,

S-(p-Cyanophenyl)-2-hexyl-1,2,3,4-tetrahydro-thio-6-naphthoat,

S-(p-Cyanophenyl)-2-heptyl-1,2,3,4-tetrahydro-thio-6-naphthoat,

S-(p-Aethoxyphenyl)-2-pentyl-1,2,3,4-tetrahydro-thio-6-naphthoat,

(trans-4-Cyano-1-cyclohexyl)-2-propyl-1,2,3,4-tetrahydro-6-naphthoat,

(trans-4-Cyano-1-cyclohexyl)-2-butyl-1,2,3,4-tetrahydro-6-naphthoat,

(trans-4-Cyano-1-cyclohexyl)-2-pentyl-1,2,3,4-tetrahydro-6-naphthoat,

(trans-4-Cyano-1-cyclohexyl)-2-hexyl-1,2,3,4-tetrahydro-6-naphthoat,

(trans-4-Cyano-1-cyclohexyl)-2-heptyl-1,2,3,4-tetrahydro-6-naphthoat,

(trans-4-Methyl-1-cyclohexyl)-2-propyl-1,2,3,4-tetra-

hydro-6-naphthoat,
(trans-4-Methyl-1-cyclohexyl)-2-pentyl-1,2,3,4-tetrahydro-6-naphthoat,
(trans-4-Methyl-1-cyclohexyl)-2-heptyl-1,2,3,4-tetrahydro-6-naphthoat,
(trans-4-Propyl-1-cyclohexyl)-2-propyl-1,2,3,4-tetrahydro-6-naphthoat,
(trans-4-Propyl-1-cyclohexyl)-2-pentyl-1,2,3,4-tetrahydro-6-naphthoat,
(trans-4-Propyl-1-cyclohexyl)-2-heptyl-1,2,3,4-tetrahydro-6-naphthoat,
2-Propyl-1,2,3,4-tetrahydro-6-naphthonitril,
2-Pentyl-1,2,3,4-tetrahydro-6-naphthonitril,
2-Heptyl-1,2,3,4-tetrahydro-6-naphthonitril,
2-Pentyloxy-1,2,3,4-tetrahydro-6-naphthonitril,
2,6-Dipropyl-1,2,3,4-tetrahydro-6-naphthalin,
2-Propyl-6-pentyl-1,2,3,4-tetrahydro-6-naphthalin,
2-Propyl-6-heptyl-1,2,3,4-tetrahydro-6-naphthalin,
2-Pentyl-6-propyl-1,2,3,4-tetrahydro-6-naphthalin,
2,6-Dipentyl-1,2,3,4-tetrahydro-6-naphthalin,
2-Pentyl-6-heptyl-1,2,3,4-tetrahydro-6-naphthalin,
2-Heptyl-6-propyl-1,2,3,4-tetrahydro-6-naphthalin,
2-Heptyl-6-pentyl-1,2,3,4-tetrahydro-6-naphthalin,
2-Propyloxy-6-pentyl-1,2,3,4-tetrahydro-6-naphthalin,
2-Pentyloxy-6-pentyl-1,2,3,4-tetrahydro-6-naphthalin.

Die Verbindungen der Formel I können erfindungsgemäss dadurch hergestellt werden, dass man

a) zur Herstellung der Verbindungen der Formel I, worin $R^2$ eine Estergruppe der Formel II bedeutet, eine Verbindung der allgemeinen Formel

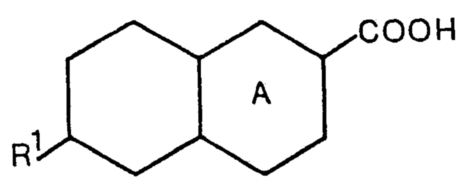

III

worin $R^1$ und A die obige Bedeutung haben, oder ein reaktionsfähiges Derivat hiervon mit einer Verbindung der allgemeinen Formel

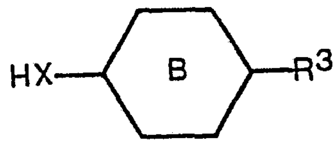

IV

worin X, B und $R^3$ die obige Bedeutung haben, oder einem geeigneten Salz hiervon verestert,

b) zur Herstellung der Verbindungen der Formel I, worin $R^2$ Cyano bedeutet, eine Verbindung der allgemeinen Formel

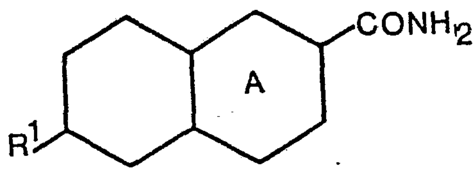

V

worin $R^1$ und A die obige Bedeutung haben, dehydratisiert,

c) zur Herstellung der Verbindungen der Formel I, worin $R^2$ eine geradkettige Alkylgruppe mit 1 bis 11 Kohlenstoffatomen bedeutet, eine Verbindung der allgemeinen Formel

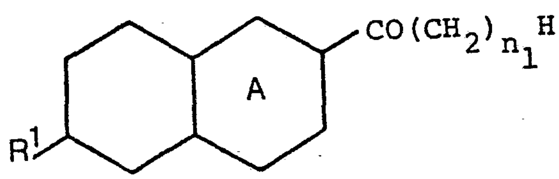

VI

worin $n_1$ eine ganze Zahl von 0 bis 10 bezeichnet und $R^1$ und A die obige Bedeutung haben, mit Hydrazin in Gegenwart einer Base umsetzt,

d) zur Herstellung der Verbindungen der Formel I, worin $R^2$ eine geradkettige Alkylgruppe mit 2 bis 11 Kohlenstoffatomen bedeutet, eine Verbindung der allgemeinen Formel

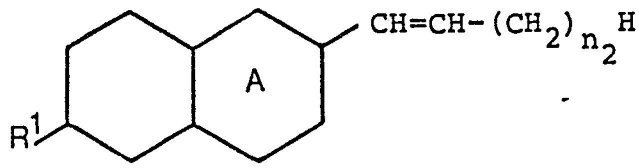

VII

worin $n_2$ eine ganze Zahl von 0 bis 9 bezeichnet und $R^1$ und A die obige Bedeutung haben, katalytisch hydriert,

e) zur Herstellung der Verbindungen der Formel I, worin Ring A gesättigt ist und $R^2$ eine geradkettige Alkoxygruppe darstellt, eine Verbindung der allgemeinen Formel

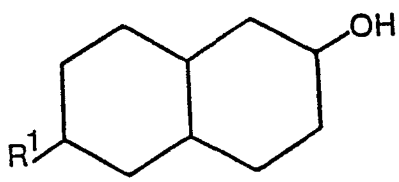

VIII

worin $R^1$ die obige Bedeutung hat, veräthert.

Die Veresterung einer Verbindung der Formel III oder eines reaktionsfähigen Derivates, beispielsweise eines Anhydrides oder Säurehalogenides, mit einer Verbindung der Formel IV oder einem geeigneten Salz hiervon, beispielsweise dem Natriumsalz, kann in an sich bekannter Weise erfolgen. Die Reaktion einer Säure der Formel III mit einer Verbindung der Formel IV wird zweckmässig in

Gegenwart einer katalytischen Menge Säure, beispielsweise Schwefelsäure oder Halogenwasserstoffsäure, mit oder ohne inertes organisches Lösungsmittel durchgeführt. Sie kann aber auch in Gegenwart von N,N'-Dicyclohexyl-carbodiimid und 4-(Dimethylamino)pyridin erfolgen. Die bevorzugte Methode ist jedoch die Umsetzung des Säurechlorids (Formel IX in Schema 3) einer Verbindung der Formel III mit einer Verbindung der Formel IV. Diese Umsetzung wird zweckmässig in einem inerten organischen Lösungsmittel, beispielsweise einem Aether, wie Diäthyläther oder Tetrahydrofuran, oder Dimethylformamid, Benzol, Toluol, Cyclohexan, Tetrachlorkohlenstoff und dergleichen durchgeführt. Um den bei der Reaktion frei werdenden Chlorwasserstoff zu binden, benützt man zweckmässig ein Säurebindemittel, beispielsweise tertiäre Amine, Pyridine und dergleichen. Vorzugsweise wird das Säurebindemittel in grossem Ueberschuss verwendet, sodass es gleichzeitig als Lösungsmittel dienen kann. Temperatur und Druck sind nicht kritisch und im allgemeinen wird diese Reaktion bei Atmosphärendruck und einer Temperatur zwischen Raumtemperatur und der Siedetemperatur des Reaktionsgemisches durchgeführt.

Die Dehydratisierung einer Verbindung der Formel V kann mit irgendeinem geeigneten Dehydratisierungsmittel wie z.B. mit Phosphoroxychlorid, Phosphorpentoxid, Thionylchlorid, Acetanhydrid oder insbesondere Benzolsulfochlorid und dergleichen durchgeführt werden. Die Dehydratisierung kann in einem inerten organischen Lösungsmittel, wie beispielsweise einem Kohlenwasserstoff oder Halogenkohlenwasserstoff, gegebenenfalls in Gegenwart einer Base, wie Natriumacetat, Pyridin oder Triäthylamin, erfolgen. Sie kann jedoch auch ohne organische Lösungsmittel durchgeführt werden. Die Reaktionstemperatur liegt vorzugsweise zwischen etwa 50°C und der Rückflusstemperatur des Reaktionsgemisches. Der Druck ist nicht kritisch und die Reaktion wird mit Vorteil bei Atmosphärendruck durchgeführt.

Die Umsetzung einer Verbindung der Formel VI mit Hydrazin in Gegenwart einer Base, beispielsweise Kaliumhydroxid, Natriumäthylat, Kalium-tert-butylat und dergleichen, erfolgt zweckmässig in einem inerten organischen Lösungsmittel wie z.B. Dimethylsulfoxid oder einem Alkohol, beispielsweise Aethanol, Di- oder Triäthylenglykol und dergleichen. Im allgemeinen wird das gebildete Hydrazon erst bei erhöhter Temperatur, beispielsweise etwa 200°C, zersetzt. Wird jedoch Dimethylsulfoxid als Lösungsmittel verwendet, so erfolgt die Zersetzung häufig schon bei Raumtemperatur. Liegt die Zersetzungstemperatur über der Siedetemperatur des Reaktionsgemisches, so muss unter erhöhtem Druck gearbeitet werden. Die bevorzugte Methode ist die Umsetzung nach dem Huang-Minlon-Verfahren, d.h. Erhitzen eines Ketons der Formel VI unter Rückfluss in einem hochsiedenden, mit Wasser mischbaren Lösungsmittel, beispielsweise Di- oder Triäthylenglykol, zusammen mit Hydrazinhydrat und Kaliumhydroxid, anschliessendes Abdestillieren des Wassers bis zur Zersetzung des Hydrazons, und weiteres Kochen unter Rückfluss bis die Reduktion beendet ist.

Die katalytische Hydrierung einer Verbindung der Formel VII kann mit irgendeinem üblichen Hydrierkatalysator, wie Palladium, Platin, Raney-Nickel und dergleichen, gegebenenfalls auf einem inerten Trägermaterial, durchgeführt werden. Bevorzugte Katalysatoren sind Palladium und Platin. Als Lösungsmittel können irgendwelche inerte organische Lösungsmittel, wie gesättigte Alkohole, Aether, Ester, Carbonsäuren und dergleichen, beispielsweise Aethanol, Dioxan, Essigsäure-äthylester oder Eisessig, verwendet werden. Zweckmässigerweise wird mit Zusatz einer Base, wie Triäthylamin oder 4-(Dimethylamino)pyridin, gearbeitet. Temperatur und Druck sind keine kritischen Aspekte in dieser Reaktion. Zweckmässigerweise werden eine Temperatur zwischen Raumtemperatur und der Siedetemperatur des Reaktionsgemisches und ein Druck von etwa 1 bis etwa 5 Atmosphären angewendet.

Zur Verätherung einer Verbindung der Formel VIII wird zweckmässig ein entsprechendes Alkoholat, z.B. das Natriumalkoholat, mit einem entsprechenden Alkylhalogenid, vorzugsweise dem Alkylbromid oder -jodid, umgesetzt. Das Alkoholat kann in an sich bekannter Weise erhalten werden, z.B. durch Umsetzung des Alkohols mit einem Alkalimetall oder Alkalimetallhydrid. Die Verätherung wird zweckmässig in einem inerten organischen Lösungsmittel, beispielsweise einem Kohlenwasserstoff oder Aether durchgeführt. Eine bevorzugte Variante dieses Verfahrens ist die Umsetzung eines Alkohols der Formel VIII mit Natriumhydrid und einem Alkylbromid oder -jodid in Monoglym, gegebenenfalls in Gegenwart von Natriumjodid. Temperatur und Druck sind nicht kritisch. Die Reaktion wird jedoch bevorzugt bei Atmosphärendruck und Raumtemperatur durchgeführt.

Die Verbindungen der Formel IV sind bekannte oder Analoge bekannter Verbindungen. Die Verbindungen der Formeln III und V-IX sind neu und bilden ebenfalls Gegenstand der vorliegenden Erfindung. Die Säuren der Formel III besitzen zum grössten Teil flüssigkristalline Eigenschaften. Die Verbindungen der Formeln III, V, VI und IX können zusammengefasst werden durch die allgemeine Formel

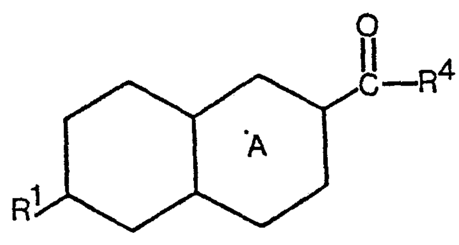

X

worin $R^1$ und A die obige Bedeutung haben und $R^4$ Wasserstoff, Chlor, die Hydroxy- oder Aminogruppe oder eine geradkettige Alkylgruppe mit 1 bis 10 Kohlenstoffatomen darstellt.

Die neuen Verbindungen der Formeln III und V-IX können nach den folgenden Reaktionsschemata 1-3, worin $R^1$, A, $n_1$ und $n_2$ die obige Bedeutung haben, $n_3$ eine ganze Zahl

von 1 bis 10 bezeichnet, das Symbol (∿) angibt, dass der betreffende Substituent in α- oder β-Stellung (unter oder über der Zeichenebene) stehen kann, und die unterbrochene Linie (-----) angibt, dass eine der bezeichneten Bindungen eine Doppelbindung ist, hergestellt werden.

Schema 1

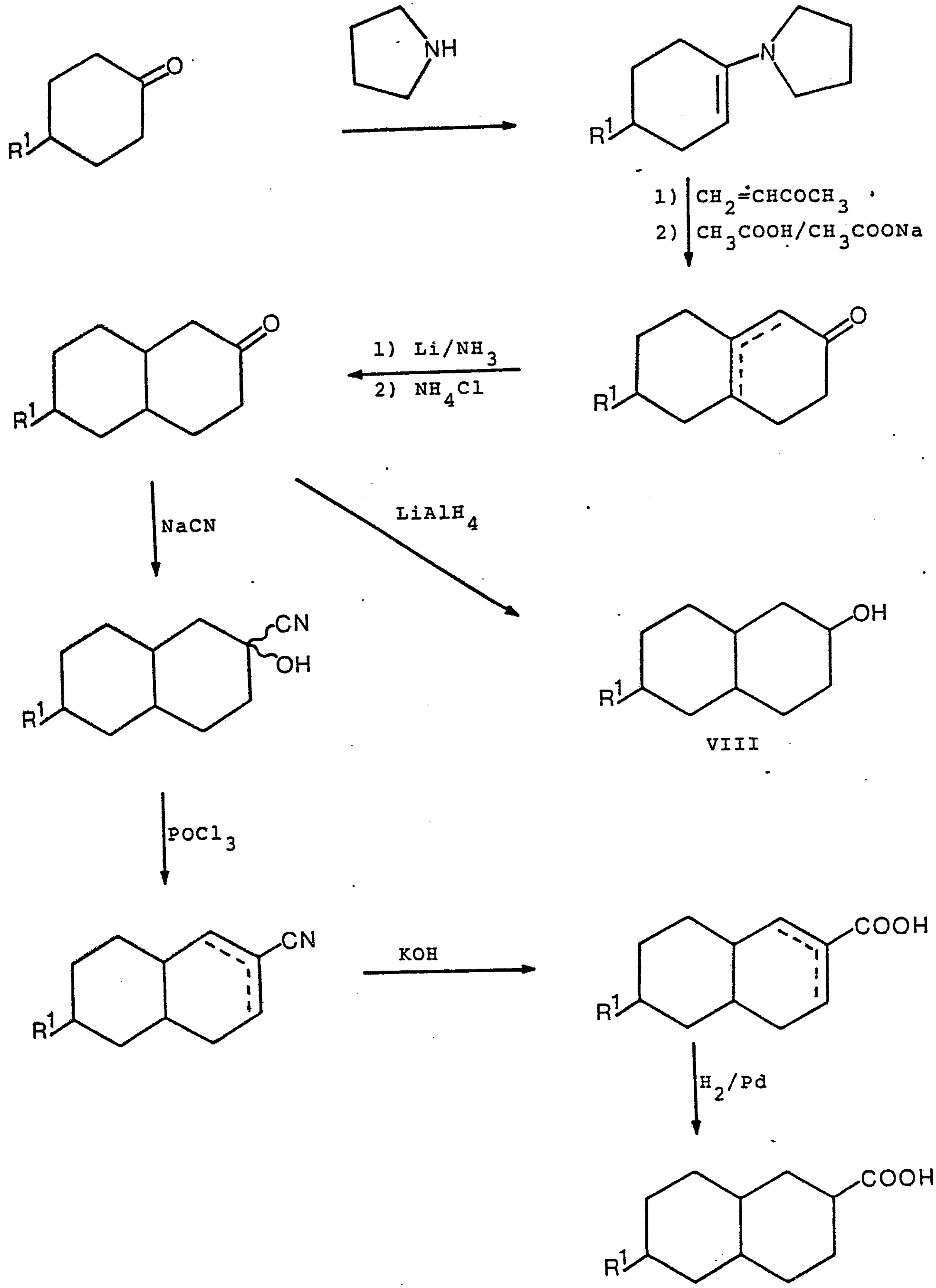


VIII

III A

Schema 2

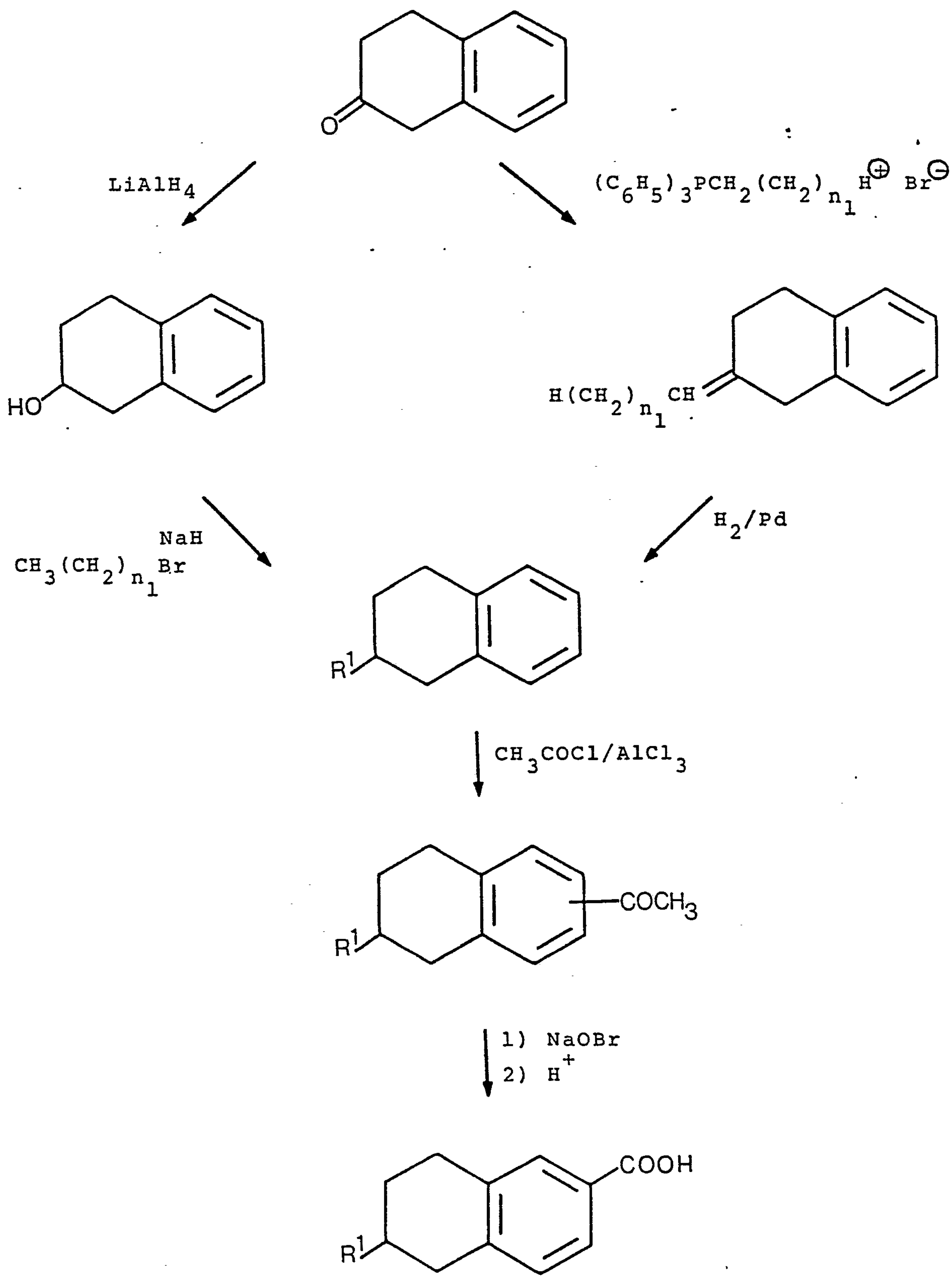


III B

Schema 3

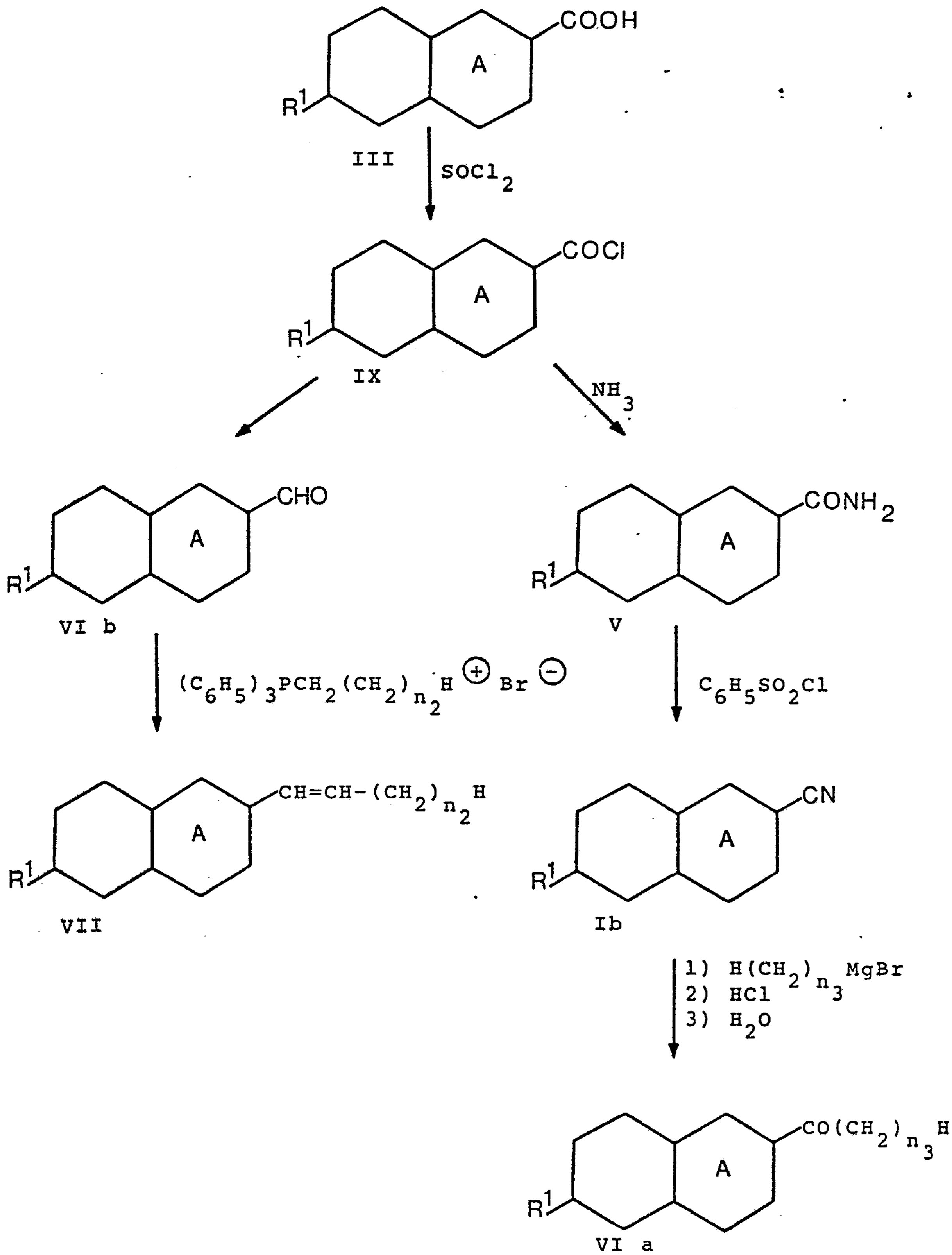


III

IX

VI b

V

VII

Ib

VI a

Die Umsetzung eines Cyclohexanons mit Pyrrolidin (gemäss Schema 1) kann beispielsweise in Toluol mit einer katalytischen Menge p-Toluolsulfonsäure erfolgen. Das gebildete Wasser wird zweckmässigerweise mit Hilfe eines Wasserabscheiders abgetrennt.

Die Umsetzung des 1-Pyrrolidinyl-1-cyclohexens mit Methylvinylketon erfolgt am geeignetsten in absolutem Toluol und Inertgasatmosphäre durch Zutropfen des Methylvinylketons und, nach einigen Stunden, Erhitzen zum Rückfluss unter Zutropfen einer Natriumacetat/Essigsäure-Lösung. Dabei wird ein Gemisch von 6-Pentyl-octahydro-$\Delta^{1,9}$-naphthalin-2-on und 6-Pentyl-octahydro-$\Delta^{9,10}$-naphthalin-2-on erhalten, das ohne Trennung weiter verwendet werden kann.

Die Reduktion des Gemisches der ungesättigten Ketone kann beispielsweise mit Lithium in flüssigem Ammoniak durchgeführt werden. Nach Zugabe von Ammoniumchlorid und Aufarbeitung erhält man das entsprechende trans-Decalin-2-on im Gemisch mit dem cis-Decalin-2-on.

Das Decalin-2-on-Gemisch, gelöst in einem inerten organischen Lösungsmittel z.B. Aether, kann beispielsweise mit einer Natriumcyanid-Lösung und Salzsäure bei etwa 0°C in das entsprechende Cyanhydrin-Gemisch übergeführt werden.

Die Dehydratisierung des Cyanhydrin-Gemisches kann mit irgendeinem geeigneten Dehydratisierungsmittel, wie Phosphoroxychlorid, Phosphorpentoxid und dergleichen, in einem inerten organischen Lösungsmittel oder Lösungsmittelgemisch, vorzugsweise Pyridin, Benzol und dergleichen, und Erhitzen zum Rückfluss durchgeführt werden. Das dabei erhaltene Gemisch von Octahydro-$\Delta^{1}$- und -$\Delta^{2}$-naphthalin-2-carbonitril kann ohne Trennung weiter verarbeitet werden.

Die Verseifung des Gemisches ungesättigter Nitrile

kann beispielsweise mit einer Lösung von Kaliumhydroxid in Diäthylenglykol und erhitzen auf etwa 200°C durchgeführt werden. Nach Ansäuern und Extraktion wird ein Gemisch der entsprechenden Octahydro-$\Delta^1$- und -$\Delta^2$-naphthalincarbonsäuren erhalten.

Die katalytische Hydrierung der ungesättigten Säuren kann mit irgendeinem üblicherweise verwendeten Hydrierkatalysator, wie Palladium, Platin, Raney-Nickel und dergleichen, gegebenenfalls auf einem inerten Trägermaterial, durchgeführt werden. Als Lösungsmittel kommen gesättigte Alkohole, Aether, Ester, Carbonsäuren und dergleichen, vorzugsweise Aethanol, in Frage. Die erhaltene rohe trans-Decalin-2-carbonsäure der Formel IIIA, die meist noch cis-Isomeres enthält, kann durch Umkristallisation und Sublimation im Hochvakuum gereinigt werden.

Die Umsetzung des oben beschriebenen trans-Decalin-2-ons, gegebenenfalls im Gemisch mit dem cis-Isomeren, zu einem Alkohol der Formel VIII kann beispielsweise mit Lithiumaluminiumhydrid in einem inerten organischen Lösungsmittel, wie Tetrahydrofuran, Diäthyläther und dergleichen, durchgeführt werden. Das erhaltene Rohprodukt der Formel VIII kann durch Umkristallisation und Chromatographie gereinigt werden.

2-Tetralon kann (gemäss Schema 2) beispielsweise durch Wittig-Alkylierung mit einem Alkyltriphenylphosphoniumbromid zum entsprechenden 2-Alkyliden-1,2,3,4-tetrahydronaphthalin umgesetzt werden. Die Reaktion wird zweckmässig in einem inerten organischen Lösungsmittel, beispielsweise einem Aether oder einem gesättigten oder aromatischen Kohlenwasserstoff, wie Diäthyläther, Tetrahydrofuran, Dioxan, Dimethoxyäthan, Toluol und dergleichen, bei einer Temperatur zwischen Raumtemperatur und Siedetemperatur des Reaktionsgemisches durchgeführt. Als Basen werden bevorzugt Alkalimetallalkoholate, wie Kalium-tert-butylat und Natriummethylat, eingesetzt.

Die katalytische Hydrierung eines 2-Alkyliden-1,2,3,4-tetrahydronaphthalins kann in analoger Weise zu der oben beschriebenen Hydrierung einer Verbindung der Formel VII durchgeführt werden.

Die Reduktion von 2-Tetralon zum Alkohol kann beispielsweise mit Lithiumaluminiumhydrid in einem inerten organischen Lösungsmittel, wie Tetrahydrofuran, Diäthyläther, und dergleichen, durchgeführt werden.

Die Verätherung des Alkohols (bzw. eines Alkoholates) zum 2-Alkoxy-1,2,3,4-tetrahydronaphthalin kann in analoger Weise zur oben beschriebenen Verätherung eines Alkohols der Formel VIII durchgeführt werden.

Die 2-Alkyl- bzw. 2-Alkoxy-1,2,3,4-tetrahydronaphthaline können in an sich bekannter Weise durch Friedel-Crafts-Acylierung, beispielsweise mit Acetylchlorid und Aluminiumchlorid in Dichlormethan, und Oxidation mit Natriumhypobromit, vorzugsweise in Dioxan, in ein Gemisch der entsprechenden 1,2,3,4-Tetrahydronaphthalin-6- und -7-carbonsäuren übergeführt werden. Dieses Gemisch kann durch Umkristallisation getrennt werden; einfacher ist jedoch die Trennung der entsprechenden Amide.

Die Carbonsäuren der Formel III können in an sich bekannter Weise, beispielsweise mit Thionylchlorid, zu Säurechloriden der Formel IX umgesetzt werden.

Die Umsetzung eines Säurechlorids der Formel IX zum entsprechenden Amid der Formel V kann beispielsweise mit Ammoniakgas in einem inerten organischen Lösungsmittel, z.B. einem Halogenkohlenwasserstoff wie Dichlormethan, durchgeführt werden.

Die Dehydratisierung eines Amides der Formel V zu einem Nitril der Formel Ib wurde bereits oben beschrieben.

Die Nitrile der Formel Ib können mit einer Grignard-Lösung, beispielsweise einer Lösung des entsprechenden Alkylmagnesiumbromids in einem Aether, wie Diäthyläther, Tetrahydrofuran und dergleichen, alkyliert und durch Einleiten von Chlorwasserstoff-Gas in Iminhydrochloride übergeführt werden. Diese können dann durch leichtes Erwärmen mit Wasser zu Ketonen der Formel VIa umgesetzt werden.

Die oben beschriebenen Säurechloride der Formel IX können ferner in an sich bekannter Weise, z.B. durch Rosenmund-Reaktion mit Wasserstoff und einem teilweise vergifteten Palladium-Katalysator in einem siedenden Kohlenwasserstoff, in Aldehyde der Formel VIb übergeführt werden.

Die Aldehyde der Formel VIb können schliesslich durch Wittig-Alkylierung, in analoger Weise zur oben beschriebenen Alkylierung von 2-Tetralon, zu Verbindungen der Formel VII umgesetzt werden.

Zur Herstellung optisch aktiver Verbindungen der Formel I werden zweckmässigerweise bereits die Verbindungen der Formeln III bzw. VIII nach an sich bekannten Methoden der Racematspaltung (z.B. über diastereomere Amide bzw. Ester) in die optischen Antipoden gespalten und dann in analoger Weise weiter umgesetzt. Beispielsweise können die Säuren der Formel IIIA (welche 4 chirale Zentren aufweisen) mit α-Phenyläthylamin umgesetzt, die erhaltenen diastereomeren Amide getrennt und anschliessend zur optisch aktiven Säure hydrolysiert werden.

Die Verbindungen der Formel I können als Gemische bestehend aus zwei oder mehreren Verbindungen der Formel I oder in Form ihrer Gemische mit anderen nematischen und/oder nicht-nematischen Substanzen verwendet werden, wie z.B. mit Substanzen aus den Klassen der Schiffschen Basen, Azo- oder Azoxybenzole, Phenylbenzoate, Cyclohexancarbonsäure-phenylester, Bi- und Terphenyle, Phenylcyclohexane, Zimtsäurederivate, Phenyl- und Diphenylpyrimidine, Cyclohexylphenylpyrimidine, Phenyldioxane und dergleichen. Derartige Verbindungen sind dem Fachmann geläufig und bekannt, z.B. aus den deutschen Offenlegungsschriften 2 306 738, 2 306 739, 2 429 093, 2 356 085, 2 636 684, 2 459 374, 2 547 737, 2 641 724, 2 708 276, 2 811 001 und aus Z. Naturforsch. 34b, 1535 (1979). Viele derartige nematische oder nicht-nematische Substanzen sind zudem im Handel erhältlich. Die Verbindungen der Formel I, worin $R^2$ eine Estergruppe der Formel II bedeutet, können jedoch auch in reiner Form verwendet werden.

Die erfindungsgemässen flüssigkristallinen Mischungen müssen mindestens eine Verbindung mit flüssigkristallinen Eigenschaften in ausreichender Menge enthalten, so dass auch die Gesamtmischung flüssigkristalline Eigenschaften besitzt. Das Gewichtsverhältnis der Mischungskomponenten entspricht vorzugsweise der eutektischen Zusammensetzung. Der Anteil der Verbindungen der Formel I kann jedoch, sofern $R^2$ eine Estergruppe der Formel II bedeutet, im allgemeinen beliebig gewählt werden und vorzugsweise etwa 1 bis etwa 80 Molprozente betragen und, sofern $R^2$ Alkyl, Alkoxy oder Cyano bedeutet, in der Regel bis zu etwa 40 Molprozenten, vorzugsweise zwischen etwa 1 und etwa 30 Molprozenten betragen.

Die erfindungsgemässen Verbindungen können ferner optisch-aktive Verbindungen, beispielsweise optisch-aktive Biphenyle, und/oder dichroitische Farbstoffe, beispielsweise Azo-, Azoxy- und Anthrachinon-Farbstoffe, enthalten. Der Anteil solcher Verbindungen wird durch die gewünschte Ganghöhe (pitch), Farbe, Extinktion, Löslichkeit und dergleichen bestimmt.

Die Herstellung von Gemischen, welche u.a. Verbindungen der Formel I sowie andere flüssigkristalline und/ oder nicht-flüssigkristalline Verbindungen und/oder dichroitische Farbstoffe enthalten, kann in an sich bekannter Weise erfolgen, z.B. durch Erhitzen einer Mischung der

Komponenten auf eine Temperatur knapp oberhalb des Klärpunktes und anschliessendes Abkühlen.

Die Erfindung betrifft ferner alle neuen Verbindungen, Mischungen, Verfahren, Verwendungen und Vorrichtungen wie hierin beschrieben.

Als Beispiele von bevorzugten nematischen Gemischen können die folgenden Mischungsbeispiele 1-13 genannt werden. Die Mischungsbeispiele 1-10 veranschaulichen den Einfluss der erfindungsgemässen Verbindungen, wenn diese zu Mischungen bekannter Flüssigkristalle (Basismischungen A-C) zugesetzt werden. $\eta_M/\eta_H$ ist das Verhältnis der Viskosität einer Mischung ($\eta_M$) zur Viskosität der verwendeten Basismischung ($\eta_H$).

Basismischung A

5,77 Gew.-% p-Butylbenzoesäure-p'-cyanophenylester,
5,19 Gew.-% p-(5-Pentyl-2-pyrimidinyl)benzonitril,
11,54 Gew.-% p-(5-Heptyl-2-pyrimidinyl)benzonitril,
12,15 Gew.-% trans-4-Propylcyclohexancarbonsäure-p'-cyanophenylester,
12,37 Gew.-% trans-4-Pentylcyclohexancarbonsäure-p'-cyanophenylester,
22,02 Gew.-% trans-4-Butylcyclohexancarbonsäure-p-(äthoxy)-phenylester,
19,92 Gew.-% trans-4-Pentylcyclohexancarbonsäure-p-(methoxy)-phenylester,
11,04 Gew.-% trans-p-[5-(4-Aethylcyclohexyl)-2-pyrimidinyl]-benzonitril;
Smp. < -10°C, Klp. 72,7-73,0°C.

Basismischung B

8,00 Gew.-% p-Butylbenzoesäure-p'-cyanophenylester,
7,19 Gew.-% p-(5-Pentyl-2-pyrimidinyl)benzonitril,
15,00 Gew.-% p-(5-Heptyl-2-pyrimidinyl)benzonitril,
15,54 Gew.-% trans-4-Propylcyclohexancarbonsäure-p-cyano-

phenylester,
9,71 Gew.-% trans-4-Butylcyclohexancarbonsäure-p-cyanophenylester,
15,54 Gew.-% trans-4-Pentylcyclohexancarbonsäure-p-cyanophenylester,
12,52 Gew.-% trans-p-[5-(4-Aethylcyclohexyl)-2-pyrimidinyl]-benzonitril,
16,50 Gew.-% trans-p-[5-(4-Heptylcyclohexyl)-2-pyrimidinyl]-benzonitril;
Smp.< -10°C, Klp. 104,6-106,8°C.

Basismischung C

28,34 Gew.-% 4'-Pentyl-4-cyanobiphenyl,
7,94 Gew.-% trans-4-Propylcyclohexancarbonsäure-p-cyanophenylester,
7,22 Gew.-% trans-4-Pentylcyclohexancarbonsäure-p-cyanophenylester,
14,68 Gew.-% trans-4-Butylcyclohexancarbonsäure-p-(äthoxy)-phenylester,
13,11 Gew.-% trans-4-Pentylcyclohexancarbonsäure-p-(methoxy)-phenylester,
8,03 Gew.-% trans-p-[5-(4-Aethylcyclohexyl)-2-pyrimidinyl]-benzonitril,
5,74 Gew.-% trans-p-[5-(4-Pentylcyclohexyl)-2-pyrimidinyl]-benzonitril,
14,94 Gew.-% trans-p-[5-(4-Heptylcyclohexyl)-2-pyrimidinyl]-benzonitril;
Smp.< -10°C, Klp. 97,5°C.

Mischungsbeispiel 1

90,0 Gew.-% Basismischung A,
10,0 Gew.-% 6-Butyl-trans-decalin-2-carbonsäure-trans-4-pentyl-1-cyclohexylester;
Smp.< -10°C, Klp. 75,4-75,7°C.

Mischungsbeispiel 2

87,53 Gew.-% Basismischung A,
12,47 Gew.-% 6-Pentyl-trans-decalin-2-carbonsäure-p-äthoxyphenylester;
Smp. < -10°C, Klp. 80,7-81,1°C.

Mischungsbeispiel 3

87,47 Gew.-% Basismischung A,
12,53 Gew.-% p-Cyanophenyl-2-heptyl-1,2,3,4-tetrahydro-6-naphthoat;
Smp. < -10°C, Klp. 79,7-80,2°C.

Mischungsbeispiel 4

86,87 Gew.-% Basismischung A,
13,13 Gew.-% p-Aethoxyphenyl-2-heptyl-1,2,3,4-tetrahydro-6-naphthoat;
Smp. < -10°C, Klp. 78,8-79,1°C.

Mischungsbeispiel 5

87,33 Gew.-% Basismischung A,
12,67 Gew.-% p-Butylphenyl-2-pentyl-1,2,3,4-tetrahydro-6-naphthoat;
Smp. < -10°C, Klp. 73,3-73,6°C.

Mischungsbeispiel 6

91,73 Gew.-% Basismischung A,
8,27 Gew.-% 2-Aethyl-6-pentyl-trans-decalin;
Smp. < -10°C, Klp. 61,7-62,5°C; $\eta_M/\eta_H$ = 0,783

Mischungsbeispiel 7

91,73 Gew.-% Basismischung C,
8,27 Gew.-% 2-Aethyl-6-pentyl-trans-decalin;

Smp. < -10°C, Klp. 83,4-86,2°C; $\eta_M / \eta_H$ = 0,80

Mischungsbeispiel 8

91,73 Gew.-% Basismischung B,
8,27 Gew.-% 2-Aethyl-6-pentyl-trans-decalin;
Smp. < -10°C, Klp. 89,7-91,9°C; $\eta_M / \eta_H$ = 0,63

Mischungsbeispiel 9

90,80 Gew.-% Basismischung A,
9,20 Gew.-% 2-Aethyl-6-heptyl-trans-decalin;
Smp. < -10°C, Klp. 62,0-62,7°C; $\eta_M / \eta_H$ = 0,771

Mischungsbeispiel 10

88,87 Gew.-% Basismischung A,
11,13 Gew.-% 6-Propyl-trans-decalin-2-carbonsäure-p-äthylphenylester;
Smp. < -10°C, Klp. 74,0-74,4°C

Mischungsbeispiel 11

8,00 Gew.-% p-Butylbenzoesäure-p'-cyanophenylester,
7,20 Gew.-% p-(5-Pentyl-2-pyrimidinyl)benzonitril,
16,00 Gew.-% p-(5-Heptyl-2-pyrimidinyl)benzonitril,
29,07 Gew.-% trans-4-Butylcyclohexancarbonsäure-p-(äthoxy)-phenylester,
6,22 Gew.-% 6-Propyl-trans-decalin-2-carbonsäure-p-cyanophenylester,
13,62 Gew.-% 6-Pentyl-trans-decalin-2-carbonsäure-p-äthylphenylester,
19,89 Gew.-% 6-Butyl-trans-decalin-2-carbonsäure-trans-4-pentyl-1-cyclohexylester;
Smp. < -10°C, Klp. 70,4-70,5°C.

Mischungsbeispiel 12

22,97 Gew.-% 4'-Heptyl-4-cyanobiphenyl,
24,24 Gew.-% trans-4-Butylcyclohexancarbonsäure-p-(äthoxy)-phenylester,
21,33 Gew.-% trans-4-Pentylcyclohexancarbonsäure-p-(methoxy)-phenylester,
5,18 Gew.-% 6-Propyl-trans-decalin-2-carbonsäure-p-cyano-phenylester,
11,35 Gew.-% 6-Pentyl-trans-decalin-2-carbonsäure-p-äthyl-phenylester,
14,93 Gew.-% 6-Butyl-trans-decalin-2-carbonsäure-trans-4-pentyl-1-cyclohexylester;
Smp. <-10°C, Klp. 70,6-70,7°C.

Mischungsbeispiel 13

22,19 Gew.-% trans-4-Butylcyclohexancarbonsäure-p-(äthoxy)-phenylester,
20,18 Gew.-% trans-4-Pentylcyclohexancarbonsäure-p-(methoxy)-phenylester,
29,63 Gew.-% p-[2-(trans-4-Propylcyclohexyl)-1-äthyl]benzonitril,
4,31 Gew.-% 6-Propyl-trans-decalin-2-carbonsäure-p-cyano-phenylester,
9,45 Gew.-% 6-Pentyl-trans-decalin-2-carbonsäure-p-äthyl-phenylester,
14,24 Gew.-% 6-Butyl-trans-decalin-2-carbonsäure-trans-4-pentyl-1-cyclohexylester;
Smp. <-10°C, Klp. 64,2-64,3°C.

Die Herstellung der erfindungsgemässen Verbindungen der Formel I wird anhand der folgenden Beispiele veranschaulicht.

Beispiel 1

6,16 g (25 mMol) 2-Pentyl-1,2,3,4-tetrahydronaphthalin-6-carbonsäure werden mit 62,5 ml Thionylchlorid unter Feuchtigkeitsausschluss 2 Stunden am Rückfluss gekocht. Nach Entfernen des überschüssigen Thionylchlorids wird das Säurechlorid als gelbliches Oel erhalten.

Zu einer auf 3°C gekühlten Lösung von 2,99 g (25 mMol) p-Cyanophenol in 15 ml absolutem Pyridin tropft man unter Rühren bei 3-7°C das mit 10 ml absolutem Benzol verdünnte Säurechlorid, lässt das Reaktionsgemisch über Nacht stehen, giesst dann auf eine Mischung von 30 g Eis und 30 ml Salzsäure (1:1), extrahiert erschöpfend mit Aether und wäscht die organischen Phasen einmal mit 35 ml eiskalter 1N Natronlauge und Wasser. Nach dem Trocknen mit Natriumsulfat und Entfernen des Lösungsmittels im Vakuum wird ein kristalliner Rückstand (8,6 g) des p-Cyanophenylesters der 2-Pentyl-1,2,3,4-tetrahydronaphthalin-6-carbonsäure erhalten, der zur Reinigung an 230 g Kieselgel chromatographiert wird. Benzol/Hexan 1:1 und Benzol eluieren 8,1 g Substanz, die aus Aceton/Hexan bis zum konstanten Smp. und Klp. umkristallisiert und im Hochvakuum (0,01 mbar) bis zur Gewichtskonstanz getrocknet wird. Es werden farblose Kristalle des p-Cyanophenyl-2-pentyl-1,2,3,4-tetrahydro-6-naphthoates erhalten: Smp. 72,7-73,3°C; Klp. 127,7°C.

Die als Ausgangsmaterial verwendete 2-Pentyl-1,2,3,4-tetrahydro-naphthalin-6-carbonsäure kann wie folgt hergestellt werden:

a) Zu einer Suspension von 178 g (0,431 Mol) n-Pentyltriphenylphosphoniumbromid in 1560 ml absolutem Toluol gibt man 51,6 g (0,456 Mol) Kalium-tert-butylat, rührt 45 Minuten bei Raumtemperatur, tropft innert 50 Minuten eine Lösung von 41,9 g (0,287 Mol) 2-Tetralon in 300 ml absolutem Toluol zu und erhitzt während 3 Stunden auf 75-80°C. Man lässt abkühlen, giesst das Reaktionsgemisch auf 1500 ml Eiswasser, trennt die organische Phase ab, extrahiert die Wasserphase noch zweimal mit Toluol und wäscht die vereinigten Toluol-Phasen mit Wasser. Nach dem Trocknen über Natriumsulfat und Entfernen des Lösungsmittels im Vakuum erhält man 175 g einer bräunlichen Suspension, die zur Reinigung durch eine Säule von 450 g Kieselgel filtriert wird. Hexan und Benzol/Hexan 1:1 eluieren 53,2 g 2-Pentyliden-1,2,3,4-tetrahydronaphthalin als gelbliches Oel.

b) Eine Mischung von 53,2 g (0,268 Mol) 2-Pentyliden-1,2,3,4-tetrahydronaphthalin, 275 ml Feinsprit, 1,4 ml Triäthylamin und 1,44 g Palladium/Kohle (5%) wird bei Raumtemperatur in einer Wasserstoff-Atmosphäre geschüttelt bis die Hydrierung beendet ist (24 Stunden). Anschliessend wird vom Katalysator abfiltriert und das Lösungsmittel im Vakuum entfernt. Die als Rückstand verbleibenden 49,4 g 2-Pentyl-1,2,3,4-tetrahydronaphthalin werden zur Reinigung im Hochvakuum destilliert: 47,3 g farblose Flüssigkeit; Sdp. 106-110°C (0,5 mbar).

c) Zu einer Mischung von 47,3 g (0,234 Mol) 2-Pentyl-1,2,3,4-tetrahydronaphthalin, 22,1 g (0,281 Mol) Acetylchlorid und 380 ml absolutem Dichlormethan gibt man unter Rühren bei Raumtemperatur innert 1 Stunde portionenweise 37,6 g (0,282 Mol) wasserfreies Aluminiumchlorid, kocht die gelbbraune Reaktionsmischung während 3 Stunden unter Rückfluss, lässt über Nacht stehen und giesst dann auf ein Gemisch von 500 ml Eiswasser und 165 ml konzentrierter Salzsäure. Man trennt die organische Schicht ab, extrahiert die wässrige Phase noch zweimal mit Dichlormethan, wäscht die organischen Phasen mit 230 ml 3N Natronlauge und mit Wasser, trocknet über Natriumsulfat und entfernt das Lösungsmittel im Vakuum. Man erhält 57,8 g eines Gemisches von 2-Pentyl-6-acetyl- und 2-Pentyl-7-acetyl-1,2,3,4-tetrahydronaphthalin als bräunliche Flüssigkeit, das direkt weiter umgesetzt wird.

d) Eine Lösung von 54,6 g (0,223 Mol) des Gemisches von 2-Pentyl-6-acetyl- und 2-Pentyl-7-acetyl-1,2,3,4-tetrahydronaphthalin in 450 ml Dioxan wird auf 60°C erwärmt und unter Rühren innert 30 Minuten zu einer Lösung von Natriumhypobromit (hergestellt aus 261 ml 28%-iger Natronlauge, 202 g Eis und 112 ml Wasser durch Zutropfen von 51,5 ml Brom bei 0°C innert 35 Minuten) zufliessen gelassen. Das bräunliche Reaktionsgemisch wird anschliessend auf 30°C erwärmt, wobei unter Entfärbung die exotherme Reaktion einsetzt. Man lässt 1 Stunde nachreagieren, reduziert das überschüssige Hypobromit durch Zugabe von Natriumhydrogensulfit-Lösung, gibt 102 ml konzentrierte Salzsäure zu und extrahiert mit Dichlormethan. Nach Waschen mit Wasser, Trocknen mit Natriumsulfat und Abdampfen des Lösungsmittels im Vakuum erhält man 57,9 g eines rohen Gemisches von 2-Pentyl-1,2,3,4-tetrahydronaphthalin-6- und -7-carbonsäure als gelblichen kristallinen Rückstand. Das Gemisch der beiden Säuren kann durch wiederholte Umkristallisation aus Hexan, Aethanol, Isopropanol und dergleichen getrennt werden. Besser gelingt jedoch die Trennung der entsprechenden Amide (Herstellung gemäss Beispiel 10) durch Umkristallisation aus geeigneten Lösungsmitteln, z.B. aus Aceton und anschliessende Hydrolyse, beispielsweise mit Kaliumhydroxid in Diäthylenglykol. 2-Pentyl-1,2,3,4-tetrahydronaphthalin-6-carboxamid schmilzt bei 166,4-167,8°C und 2-Pentyl-1,2,3,4-tetrahydronaphthalin-7-carboxamid bei 112,9-123,5°C. Die flüssigkristalline 2-Pentyl-1,2,3,4-tetrahydronaphthalin-6-carbonsäure zeigt einen Schmelzpunkt von 122,9-123,1°C und einen Klärpunkt von 174,9-177,6°C; die 2-Pentyl-1,2,3,4-tetrahydronaphthalin-7-carbonsäure schmilzt bei 103,6-105,5°C und ist nicht flüssigkristallin.

In analoger Weise können folgende Verbindungen hergestellt werden:

p-Cyanophenyl-2-heptyl-1,2,3,4-tetrahydro-6-naphthoat; Smp. 73,4°C, Klp. 123,5°C

2-Heptyl-1,2,3,4-tetrahydronaphthalin-6-carbonsäure; Smp. 126,0-126,5°C, Klp. 168,4-169,5°C

2-Heptyl-1,2,3,4-tetrahydronaphthalin-7-carbonsäure; Smp. 118,4-119,0°C.

2-Heptyl-1,2,3,4-tetrahydronaphthalin-6-carboxamid; Smp. 133,5-133,9°C

2-Heptyl-1,2,3,4-tetrahydronaphthalin-7-carboxamid; Smp. 113,2-113,7°C.

## Beispiel 2

6,16 g (25 mMol) 2-Pentyl-1,2,3,4-tetrahydronaphthalin-6-carbonsäure werden analog zu Beispiel 1 in das Säurechlorid übergeführt und dann mit 3,45 g (25 mMol) p-Aethoxyphenol in 15 ml absolutem Pyridin umgesetzt. Man erhält 8,9 g rohen p-Aethoxyphenylester der 2-Pentyl-1,2,3,4-tetrahydronaphthalin-6-carbonsäure, der zur Reinigung an 230 g Kieselgel chromatographiert wird. Toluol/Hexan 1:1 und Toluol/Hexan 7:3 eluieren 8,8 g Substanz, die aus Aceton/Hexan bis zum konstanten Smp. und Klp. umkristallisiert und im Hochvakuum (0,01 mbar) getrocknet werden. Es werden farblose Kristalle von p-Aethoxyphenyl-2-pentyl-1,2,3,4-tetrahydro-6-naphthoat erhalten: Smp. 95,4-96,2°C; Klp. 119,0-119,4°C.

In analoger Weise kann folgende Verbindung hergestellt werden:

p-Aethoxyphenyl-2-heptyl-1,2,3,4-tetrahydro-6-naphthoat; Smp. 83,8°C, Klp. 116°C.

## Beispiel 3

2,0 g (8,12 mMol) 2-Pentyl-1,2,3,4-tetrahydronaphthalin-6-carbonsäure werden analog zu Beispiel 1 mit 20 ml Thionylchlorid in das Säurechlorid übergeführt und dann mit 1,234 g (8,00 mMol) p-Aethoxy-thiophenol in 5 ml absolutem Pyridin umgesetzt. Man erhält 2,9 g rohen p-Aethoxythiophenylester der 2-Pentyl-1,2,3,4-tetrahydronaphthalin-6-carbonsäure, der zur Reinigung an 95 g Kieselgel chromatographiert wird. Hexan/Toluol und Toluol eluieren 2,7 g Substanz, die aus Aceton/Hexan bis zum konstanten Smp. und Klp. umkristallisiert und im Hochvakuum (0,01 mbar) bis zur Gewichtskonstanz getrocknet werden. Es werden farblose Kristalle von S-(p-Aethoxyphenyl)-2-pentyl-1,2,3,4-tetrahydro-thio-6-naphthoat erhalten: Smp. 82°C, Klp. 145°C.

## Beispiel 4

2,0 g (8,12 mMol) 2-Pentyl-1,2,3,4-tetrahydronaphthalin-6-carbonsäure werden analog zu Beispiel 1 in das Säurechlorid übergeführt und dann mit 1,202 g (8,0 mMol) p-Butylphenol in 5 ml absolutem Pyridin umgesetzt. Man erhält 3,0 g rohen p-Butylphenylester der 2-Pentyl-1,2,3,4-tetrahydronaphthalin-6-carbonsäure, der zur Reinigung an 95 g Kieselgel chromatographiert wird. Hexan/Toluol 1:1 eluieren 2,8 g Substanz, die aus Aether/Hexan bis zum konstanten Smp. und Klp. umkristallisiert und im Hochvakuum (0,01 mbar) bis zur Gewichtskonstanz getrocknet werden. Es werden farblose Kristalle von p-Butylphenyl-2-pentyl-1,2,3,4-tetrahydro-6-naphthoat erhalten. Smp. 48,1°C, Klp. 80,7°C.

## Beispiel 5

1,893 g (7,50 mMol) 6-Pentyl-trans-decalin-2-carbonsäure werden mit 15 ml Thionylchlorid unter Feuchtigkeitsausschluss 2 Stunden am Rückfluss gekocht. Nach Entfernen des überschüssigen Thionylchlorids im Vakuum wird das Säurechlorid als bräunliche Flüssigkeit erhalten.

Zu einer auf 3°C gekühlten Lösung von 0,893 g (7,497 mMol) p-Cyanophenol in 7,5 ml absolutem Pyridin tropft man unter Rühren bei 3-7°C das mit 10 ml absolutem Benzol verdünnte Säurechlorid, erwärmt das Reaktionsgemisch 3,5 Stunden auf 50-55°C und lässt über Nacht bei Raumtemperatur stehen. Man giesst dann auf eine Mischung von 15 g Eis und 15 ml Salzsäure (1:1), extrahiert erschöpfend mit Aether und wäscht die organischen Phasen einmal mit 11,5 ml eiskalter 1N Natronlauge und mit Wasser. Der nach dem Trocknen mit Natriumsulfat und Entfernen des Lösungsmittels im Vakuum erhaltene kristalline Rückstand (2,6 g) des p-Cyanophenylesters der 6-Pentyl-trans-decalin-2-carbonsäure wird zur Reinigung an 90 g Kieselgel chromatographiert. Hexan/Toluol und Toluol eluieren 2,5 g Substanz, die bis zum konstanten Smp. und Klp. aus Hexan umkristallisiert und im Hochvakuum (0,01 mbar) bis zur Gewichtskonstanz getrocknet wird. Es werden farblose Kristalle des 6-Pentyl-trans-decalin-2-carbonsäure-p-cyanophenylesters erhalten: Smp. 79,9°C, Klp. 148,0°C.

Die als Ausgangsmaterial verwendete 6-Pentyl-trans-decalin-2-carbonsäure kann wie folgt hergestellt werden:

a) Ein Gemisch von 84,1 g (0,5 Mol) 4-Pentylcyclohexanon, 51,3 g (0,72 Mol) Pyrrolidin, 120 ml Toluol und 0,62 g p-Toluolsulfonsäure wird unter Vorschaltung eines Wasserabscheiders 2 Stunden zum Sieden erhitzt und das abgeschiedene, Pyrrolidin enthaltende Wasser abgetrennt. Der das 4-Pentyl-1-pyrrolidinyl-1-cyclohexen enthaltende Rückstand wird zuerst im Vakuum vom überschüssigen Toluol befreit und anschliessend im Hochvakuum destilliert: Sdp. 107-113°C (0,16 mbar), gelbliche Flüssigkeit.

b) Unter Rühren und Stickstoffbegasung werden zu einer Mischung von 98,4 g (0,444 Mol) 4-Pentyl-1-pyrrolidinyl-1-cyclohexen und 315 ml absolutem Toluol innert 1 Stunde 35,9 g (0,512 Mol) Methylvinylketon zugetropft (Temperaturanstieg auf 42°C). Man lässt über Nacht stehen, kocht dann 3 Stunden unter Rückfluss, tropft zur siedenden Mischung eine Lösung von 19,6 g (0,238 Mol) wasserfreiem Natriumacetat und 39,1 ml Eisessig in 39,1 ml Wasser und

kocht weitere 8 Stunden unter Rückfluss. Nach dem Abkühlen wird die Toluol-Schicht abgetrennt, die wässrigen Phasen werden noch zweimal mit Toluol extrahiert und die organischen Phasen werden der Reihe nach mit Wasser, 1N Salzsäure, Wasser, gesättigter Natriumhydrogencarbonat-Lösung und Wasser gewaschen und dann über Natriumsulfat getrocknet. Nach Entfernen des Lösungsmittels im Vakuum erhält man ein Gemisch von 6-Pentyl-octahydro-$\Delta^{1,9}$- und -$\Delta^{9,10}$-naphthalin-2- on als braune Flüssigkeit (106 g). Zur Reinigung wird in Hochvakuum destilliert. Ausbeute: 69,4 g gelbliche Flüssigkeit; Sdp. 127-132°C (0,22 mbar).

c) Zu einer Lösung von 17,2 g (2,479 g-Atome) Lithium-Draht in 2 Liter flüssigem Ammoniak (Trockeneis-Kühler) tropft man unter Rühren eine Lösung von 71,2 g (0,323 Mol) des oben erhaltenen Gemisches von 6-Pentyl-octahydro-$\Delta^{1,9}$- und -$\Delta^{9,10}$- naphthalin-2-on in 450 ml absolutem Aether, lässt 1 Stunde nachreagieren, verdünnt das Reaktionsgemisch mit 1 Liter absolutem Aether und gibt portionenweise 113 g (2,112 Mol) Ammoniumchlorid bis zur Entfärbung zu. Man lässt den Ammoniak über Nacht bei Raumtemperatur verdampfen, kühlt das Reaktionsgemisch mit Eis und stellt mit konzentrierter Salzsäure kongosauer. Nach Zugabe von Wasser und einer zusätzlichen Menge Aether trennt man die Aetherschicht ab, extrahiert die wässrige Phase noch zweimal mit Aether, wäscht die organischen Phasen mit Wasser und trocknet über Natriumsulfat. Nach dem Entfernen des Lösungsmittels im Vakuum erhält man 69,4 g eines Gemisches von vorwiegend 6-Pentyl-trans-decalin-2-on und 6-Pentyl-cis-decalin-2-on als braune Flüssigkeit, die roh weiter verarbeitet wird.

d) Zu 69,4 g (0,312 Mol) des Gemisches von 6-Pentyl-trans- und -cis-decalin-2-on, gelöst in 350 ml Aether, gibt man unter Stickstoffbegasung eine Lösung von 23,6 g (0,482 Mol) Natriumcyanid in 54 ml Wasser, kühlt auf 0°C und tropft unter Rühren innert 2 Stunden 69,9 ml 25%-ige Salzsäure zu. Anschliessend wird noch 1 Stunde bei Raumtemperatur gerührt, die organische Phase abgetrennt, die wässrige Phase noch zweimal mit Aether extrahiert, die vereinigte organische Phase mit Wasser gewaschen und mit Natriumsulfat getrocknet. Das nach dem Entfernen des Lösungsmittels im Vakuum erhaltene Cyanhydrin-Gemisch (75,6 g braunes Oel) wird roh weiter umgesetzt.

e) 75,6 g (0,303 Mol) des rohen Cyanhydrin-Gemisches werden unter Rühren in 81 ml absolutem Pyridin und 67 ml absolutem Benzol gelöst, auf -2°C gekühlt, innert 20 Minuten tropfenweise mit einer Mischung von 42,0 ml (0,460 Mol) Phosphoroxychlorid und 53,4 ml absolutem Pyridin versetzt und anschliessend 4 Stunden unter Rückfluss gekocht. Der anfänglich gebildete Niederschlag löst sich beim Erwärmen, bildet sich aber beim Abkühlen über Nacht von neuem. Man giesst auf 375 g Eis, verdünnt mit Aether, trennt die Aetherschicht ab und extrahiert die wässrige Phase noch zweimal mit Aether. Die Aetherphasen werden mit Wasser gewaschen, über Natriumsulfat getrocknet und im Vakuum vom Lösungsmittel befreit. Man erhält 72,5 g eines dunkelbraunen Oels, das vorwiegend aus 6-Pentyl-trans-octahydro-$\Delta^1$- und -$\Delta^2$-naphthalin-2-carbonitril besteht, daneben aber noch die entsprechenden 9,10-cis-Verbindungen enthält. Das Gemisch wird roh weiter umgesetzt.

f) Das erhaltene Nitril-Gemisch (72,5 g, 0,313 Mol) wird mit einer heissen Lösung von 35,1 g (0,625 Mol) Kaliumhydroxid in 355 ml Diäthylenglykol während 6,5 Stunden unter Stickstoffbegasung auf 200°C Badtemperatur erhitzt. Nach dieser Zeit ist die Ammoniak-Entwicklung praktisch beendet. Man lässt abkühlen, extrahiert die alkalische, mit 500 ml Wasser verdünnte Lösung dreimal mit Aether und wäscht die organischen Phasen zweimal mit Wasser nach. Die nach Trocknen mit Natriumsulfat und Abdampfen des Aethers erhaltenen 17,2 g dunkelbraunes Oel werden verworfen. Die wässrigen Phasen (einschliesslich Waschwasser) werden mit 3N Schwefelsäure kongosauer gestellt, wobei ein Niederschlag bzw. eine Trübung eintritt. Man extrahiert erschöpfend mit Aether, wäscht die organischen Phasen mit Wasser und trocknet mit Natriumsulfat. Nach Eindampfen im Vakuum werden 57,6 g eines braunen, festen Rückstandes erhalten, der vorwiegend aus 6-Pentyl-trans-octahydro-$\Delta^1$- und -$\Delta^2$-naphthalin-2-carbonsäure besteht, daneben aber noch die entsprechenden 9,10-cis-Verbindungen enthält. Dieser Rückstand wird zur Reinigung in warmem Toluol gelöst und durch eine Säule von 300 g Kieselgel filtriert. Toluol und Toluol mit 1% bzw. 2% Aceton eluieren insgesamt 45,9 g bräunliche, kristalline Substanz, die direkt weiter umgesetzt wird.

g) Das erhaltene Gemisch ungesättigter Säuren (45,9 g) wird in 700 ml Feinsprit warm gelöst, auf Raumtemperatur gekühlt und nach dem Versetzen mit 4,3 g Palladium/Kohle (5% Palladium) in einer Wasserstoff-Atmosphäre geschüttelt, bis die Hydrierung zum Stillstand kommt (24 Stunden). Anschliessend wird der Katalysator abfiltriert und das Lösungsmittel im Vakuum abgedampft. Man erhält 45,9 g gelblichen kristallinen Rückstand, der vorwiegend aus 6-Pentyl-trans-decalin-2-carbonsäure besteht und daneben noch das cis-Isomere enthält. Zur Reinigung wird mehrmals aus Aether/Hexan oder Hexan umkristallisiert, wobei die Reinigung mittels Gaschromatogramm, Smp. und Klp. verfolgt wird. Nach Sublimation im Hochvakuum (0,01 mbar) erhält man 13,7 g reine flüssigkristalline 6-Pentyl-trans-decalin-2-carbonsäure als farblose Kristalle; Smp. 113,5-114,3°C, Klp. 165,8-167,1°C.

In analoger Weise können folgende Verbindungen hergestellt werden:

6-Aethyl-trans-decalin-2-carbonsäure-p-cyanophenylester; Smp. 62,9°C, Klp. 122°C

6-Propyl-trans-decalin-2-carbonsäure-p-cyanophenylester; Smp. 79,9°C, Klp. 147,6°C

6-Heptyl-trans-decalin-2-carbonsäure-p-cyanophenyl-

ester; Smp. 76,4°C, Klp. 141°C (monotrop smektisch unter 56,5°C)

6-Aethyl-trans-decalin-2-carbonsäure; Smp. 113,0-114,5°C, Klp. 142,2-142,9°C

6-Propyl-trans-decalin-2-carbonsäure; Smp. 123,7-124,5°C, Klp. 165,0-166,0°C

6-Butyl-trans-decalin-2-carbonsäure; Smp. 113,9-114,6°C, Klp. 159,3-162,0°C

6-Heptyl-trans-decalin-2-carbonsäure; Smp. 114,8°C, Klp. 162,0°C.

Beispiel 6

1,893 g (7,50 mMol) 6-Pentyl-trans-decalin-2-carbonsäure werden analog zu Beispiel 5 in das Säurechlorid überführt und dann mit 1,036 g (7,498 mMol) p-Aethoxyphenol in 7,5 ml absolutem Pyridin umgesetzt. Nach gleicher Reaktionsführung und Aufarbeitung wie in Beispiel 5 erhält man 2,6 g rohen, kristallinen p-Aethoxyphenylester der 6-Pentyl-trans-decalin-2-carbonsäure, der zur Reinigung an 90 g Kieselgel chromatographiert wird. Hexan/Toluol 1:1 und Toluol eluieren 2,5 g Substanz, die bis zum konstanten Smp. und Klp. aus Aceton/Hexan umkristallisiert und im Hochvakuum (0,01 mbar) bis zur Gewichtskonstanz getrocknet wird. Man erhält 6-Pentyl-trans-decalin-2-carbonsäure-p-äthoxyphenylester als farblose Kristalle; Smp. 90,0°C, Klp. 142,6°C.

Beispiel 7

1,893 g (7,50 mMol) 6-Pentyl-trans-decalin-2-carbonsäure werden analog zu Beispiel 5 in das Säurechlorid übergeführt und dann mit 0,916 g (7,498 mMol) p-Aethylphenol in 7,5 ml absolutem Pyridin umgesetzt. Nach gleicher Reaktionsführung und Aufarbeitung erhält man 2,5 g rohen, kristallinen p-Aethylphenylester der 6-Pentyl-trans-decalin-2-carbonsäure, der zur Reinigung an 90 g Kieselgel chromatographiert wird. Hexan/Toluol 1:1 eluieren 2,3 g Substanz, die bis zum konstanten Smp. und Klp. aus Aether/Hexan und Hexan umkristallisiert und im Hochvakuum (0,01 mbar) bis zur Gewichtskonstanz getrocknet wird. Man erhält 6-Pentyl-trans-decalin-2-carbonsäure-p-äthylphenylester als farblose Kristalle; Smp. 54,1°C, Klp. 95,4°C.

In analoger Weise können folgende Verbindungen hergestellt werden:

6-Methyl-trans-decalin-2-carbonsäure-p-butylphenylester; Smp. 59,5°C, Klp. 48,9°C (monotrop)

6-Aethyl-trans-decalin-2-carbonsäure-p-äthylphenylester; Smp. 56,4°C, Klp. 57,5°C

6-Propyl-trans-decalin-2-carbonsäure-p-äthylphenylester; Smp. 74,9-75,4°C, Klp. 88,0°C

6-Butyl-trans-decalin-2-carbonsäure-p-äthylphenylester; Smp. 66,9°C, Klp. 84,5°C

6-Heptyl-trans-decalin-2-carbonsäure-p-äthylphenylester; Smp. 66,0°C

6-Heptyl-trans-decalin-2-carbonsäure-p-butylphenylester; Smp. 56,4°C, Klp. 94,8°C (monotrop smektisch unter 54,4°C).

## Beispiel 8

8,95 g (35,60 mMol) 6-Pentyl-trans-decalin-2-carboxamid werden in 94 ml absolutem Pyridin suspendiert und unter Rühren mit 12,94 g (73,29 mMol) Benzolsulfochlorid versetzt. Die klar werdende Lösung wird über Nacht bei Raumtemperatur stehengelassen, dann auf eine Mischung von 190 g Eis und 180 ml Salzsäure (1:1) gegossen und erschöpfend mit Aether extrahiert. Die Aetherlösungen werden mit Wasser neutral gewaschen, über Natriumsulfat getrocknet und das Lösungsmittel im Vakuum abgedampft. Man erhält 11,0 g rohes 6-Pentyl-trans-decalin-2-carbonitril als gelbliches, später kristallisierendes Oel, das zur Reinigung an 200 g Kieselgel chromatographiert wird.

Hexan/Toluol-Gemische mit 30%, 40% und 50% Toluol eluieren 8,1 g Substanz, die bis zum konstanten Smp. aus Hexan umkristallisiert und anschliessend im Hochvakuum destilliert wird; Sdp. 115-120°C (0,02 mbar). Man erhält 6-Pentyl-trans-decalin-2-carbonitril als farblose Kristalle; Smp. 41,9°C.

Das als Ausgangsmaterial verwendete 6-Pentyl-trans-decalin-2-carboxamid kann wie folgt hergestellt werden:

31,0 g (0,123 Mol) der in Beispiel 5 erhaltenen Mutterlaugen-Säure, die neben 6-Pentyl-trans-decalin-2-carbonsäure noch das cis-Isomere enthält, werden analog zu Beispiel 5 mit 133 ml Thionylchlorid in das Säurechlorid übergeführt. Nach Entfernen des überschüssigen Thionylchlorids wird mit 100 ml absolutem Dichlormethan verdünnt und diese Lösung unter Rühren und Kühlen zu einer mit Ammoniakgas gesättigten Lösung von 465 ml absolutem Dichlormethan getropft. Man leitet noch 2,5 Stunden Ammoniakgas ein, dampft das Reaktionsgemisch im Vakuum zur Trockene ein, versetzt mit 530 ml Wasser und 500 ml Aether und rührt 30 Minuten bei Raumtemperatur. Der Niederschlag wird genutscht, mit Wasser und Aether gewaschen und getrocknet. Man erhält 8,9 g 6-Pentyl-trans-decalin-2-carboxamid, das zur Reinigung aus Dioxan umkristallisiert und im Hochvakuum (0,01 mbar) sublimiert wird; farblose Kristalle, Smp. 211,6-212,7°C. Aus den Aetherlösungen erhält man durch mehrmalige Umkristallisation (Kontrolle mittels Gaschromatogramm) weitere 1,1 g des trans-Amids und durch Umkristallisation der Mutterlaugen aus Aether das 6-Pentyl-cis-decalin-2-carboxamid als farblose Kristalle (Smp. 126,7-128,0°C).

In analoger Weise können folgende Verbindungen hergestellt werden:

6-Aethyl-trans-decalin-2-carbonitril; Smp. 34,7°C
6-Propyl-trans-decalin-2-carbonitril; Smp. 63,8°C

6-Aethyl-trans-decalin-2-carboxamid; Smp. 224,6-225,0°C

6-Propyl-trans-decalin-2-carboxamid; Smp. 225,0-226,0°C

6-Aethyl-cis-decalin-2-carboxamid; Smp. 129,5-131,5°C

6-Propyl-cis-decalin-2-carboxamid; Smp. 126,6-127,1°C.

## Beispiel 9

Ein Gemisch von 3,478 g (11,89 mMol) 6-Pentyl-2-valeryl-trans-decalin, 11,4 ml absolutem Aethanol und 1,339 g (26,75 mMol) Hydrazinhydrat wird unter Erwärmen gelöst und über Nacht stehengelassen. Nach Zugabe von 12,2 ml Diäthylenglykol und 1,8 g (32,08 mMol) Kaliumhydroxid erwärmt man am absteigenden Kühler im Verlaufe von 2 Stunden auf 200°C Badtemperatur und belässt 1,5 Stunden bei dieser Temperatur. Destillat und Rückstand werden vereinigt, mit 25 ml Wasser versetzt und mit Aether extrahiert und die organischen Phasen mit Wasser gewaschen und über Natriumsulfat getrocknet. Nach Abdampfen des Lösungsmittels im Vakuum erhält man 3,35 g rohes 2,6-Dipentyl-trans-decalin, das zur Reinigung an 90 g Kieselgel chromatographiert wird. Hexan eluiert 1,74 g Substanz, die im Hochvakuum bei 145°C/0,03 mbar destilliert wird. Man erhält farblose Kristalle von 2,6-Dipentyl-trans-decalin; Smp. 47,8°C.

Das als Ausgangsmaterial verwendete 6-Pentyl-2-valeryl-trans-decalin kann wie folgt hergestellt werden:

Durch Zutropfen einer Mischung von 2,890 g (21,09 mMol) n-Butyl-bromid und 3,5 ml absolutem Aether zu einer Suspension von 0,513 g (21,10 mg-Atome) Magnesium in 7 ml absolutem Aether wird eine Grignard-Lösung bereitet. Nach Auflösen des Magnesiums wird unter Rühren eine Lösung von 4,101 g (17,57 mMol) 6-Pentyl-trans-decalin-2-carbonitril (hergestellt nach Beispiel 8) in 7 ml absolutem Aether bei 35-37°C zugetropft, das Reaktionsgemisch 7

Stunden unter Rückfluss gekocht und über Nacht stehengelassen. Dann werden 3,326 g (0,104 Mol) Methanol zugetropft und nach 30 Minuten Rühren wird der gebildete Niederschlag abgenutscht und gut mit absolutem Aether gewaschen. Anschliessend wird während 1 Stunde unter Kühlen auf 0°C Chlorwasserstoff-Gas in das Filtrat eingeleitet und zur Trockene eingeengt. Man erhält 6,9 g Iminhydrochlorid von 6-Pentyl-2-valeryl-trans-decalin als bräunliches, trübes Oel. Dieses wird mit 50 ml Wasser während 30 Minuten auf 50°C erwärmt, wobei sich das 6-Pentyl-2-valeryl-trans-decalin ölig und später kristallin abscheidet. Man extrahiert mit Aether, wäscht die organische Phase mit Wasser, trocknet über Natriumsulfat und entfernt das Lösungsmittel im Vakuum. Es verbleiben 5,3 g bräunliches, kristallisierendes Oel, das zur Reinigung z.B. aus Hexan umkristallisiert werden kann. Dadurch erhält man farblose Kristalle von 6-Pentyl-2-valeryl-trans-decalin; Smp. 47,0-48,5°C.

In analoger Weise können folgende Verbindungen hergestellt werden:

2-Aethyl-6-propyl-trans-decalin; Sdp. 90°C/0,007 mbar
2-Aethyl-6-pentyl-trans-decalin; Sdp. 110°C/0,007 mbar
2-Aethyl-6-heptyl-trans-decalin; Sdp. 140°C/0,007 mbar
6-Aethyl-2-propionyl-trans-decalin; flüssig
6-Aethyl-2-valeryl-trans-decalin; ölig
6-Aethyl-2-heptanoyl-trans-decalin; kristallin.

## Beispiel 10

2,605 g (10,6 mMol) 2-Pentyl-1,2,3,4-tetrahydronaphthalin-6-carboxamid werden in 40 ml absolutem Pyridin suspendiert und mit 2,86 g (16,2 mMol) Benzolsulfochlorid unter Rühren versetzt. Die klar werdende Lösung wird über Nacht bei Raumtemperatur stehengelassen, dann auf eine Mischung von 50 g Eis und 40 ml konz. Salzsäure gegossen und erschöpfend mit Dichlormethan extrahiert. Die organischen Phasen werden mit Wasser neutral gewaschen, über Natriumsulfat getrocknet und das Lösungsmittel im Vakuum abgedampft. Man erhält 2,390 g rohes 2-Pentyl-1,2,3,4-tetrahydronaphthalin-6-carbonitril als gelbliches Oel, das zur Reinigung an 90 g Kieselgel chromatographiert wird. Hexan/Toluol 1:1 und Toluol eluieren 2,35 g gelbliches Oel, das bei -10°C kristallisiert. Man kristallisiert bis zum konstanten Smp. aus wenig Aethanol um, indem die Lösung auf -10°C gekühlt und die Mutterlauge von den ausgeschiedenen Kristallen z.B. durch Dekantieren, Pipettieren oder Filtration durch eine gekühlte Nutsche getrennt wird. Anschliessend wird im Hochvakuum (0,01 mbar) bis zur Gewichtskonstanz getrocknet. Man erhält farblose Kristalle (bzw. farbloses Oel) von 2-Pentyl-1,2,3,4-tetrahydronaphthalin-6-carbonitril; Smp. 18,2°C.

Das als Ausgangsmaterial verwendete 2-Pentyl-1,2,3,4-tetrahydronaphthalin-6-carboxamid kann wie folgt hergestellt werden:

7,4 g (30,0 mMol) der nach Beispiel 1 gewonnenen 2-Pentyl-1,2,3,4-tetrahydronaphthalin-6-carbonsäure werden mit 68 ml Thionylchlorid wie in Beispiel 1 in das Säurechlorid übergeführt. Nach Entfernen des überschüssigen Thionylchlorid wird mit 100 ml Dichlormethan verdünnt und diese Lösung unter Rühren und Kühlen zu einer mit Ammoniakgas gesättigten Lösung von 200 ml absolutem Dichlormethan getropft. Man leitet noch 2 Stunden weiter Ammoniakgas ein, dampft das Reaktionsgemisch im Vakuum zur Trockene, versetzt mit 200 ml Wasser, schüttelt 30 Minuten, nutscht den Niederschlag, wäscht mit Wasser und trocknet. Man erhält 7,66 g rohes 2-Pentyl-1,2,3,4-tetrahydronaphthalin-6-carboxamid, das zur Reinigung aus Dioxan umkristallisiert und im Hochvakuum (0,01 mbar) sublimiert wird: farblose Kristalle, Smp. 166,4-167,8°C.

In analoger Weise können folgende Verbindungen hergestellt werden:

2-Heptyl-1,2,3,4-tetrahydronaphthalin-6-carbonitril; Smp. 36,5°C

2-Heptyl-1,2,3,4-tetrahydronaphthalin-6-carboxamid; Smp. 133,5-133,9°C.

## Beispiel 11

2,10 g (10 mMol) 6-Aethyl-trans-decalin-2-carbonsäure (hergestellt nach Beispiel 5) werden in 30 ml absolutem Methylenchlorid gelöst und mit einer Lösung von 1,87 g (11 mMol) 4-Pentyl-trans-cyclohexanol in 20 ml absolutem Methylenchlorid versetzt. Anschliessend werden 0,20 g (1,637 mMol) 4-(Dimethylamino)pyridin und, nach Kühlen auf 5°C, unter Rühren 2,48 g (12,02 mMol) Dicyclohexylcarbodiimid in fester Form zugegeben. Nach kurzer Zeit beginnt Dicyclohexylharnstoff auszufallen. Nach 1 Stunde bei 2°C und 1,25 Stunden bei Raumtemperatur wird der Niederschlag abgenutscht und mit Methylenchlorid und Hexan nachgewaschen. Durch Abdampfen des Filtrates im Vakuum erhält man 4,80 g rohen 6-Aethyl-trans-decalin-2-carbonsäure-trans-4-pentyl-1-cyclohexylester, der zur Reinigung an 90 g Kieselgel chromatographiert wird. Hexan/Toluol 1:1 eluieren 3,22 g Substanz, die bis zum konstanten Schmelzpunkt und Klärpunkt aus Hexan umkristallisiert und im Hochvakuum (0,01 mbar) bis zur Gewichtskonstanz getrocknet wird. Man erhält 6-Aethyl-trans-decalin-2-carbonsäure-trans-4-pentyl-1-cyclohexylester als farblose Kristalle; Smp. 50,4°C, Klp. 86,3°C.

In analoger Weise kann folgende Verbindung hergestellt werden:

6-Butyl-trans-decalin-2-carbonsäure-trans-4-pentyl-1-cyclohexylester; Smp. 62,2-65,4°C, Klp. 109,3°C.

Beispiel 12

0,952 g (3,99 mMol) 6-Butyl-trans-decalin-2-carbonsäure (hergestellt nach Beispiel 5) werden zusammen mit 0,500 g (3,99 mMol) trans-4-Hydroxycyclohexancarbonitril und 0,081 g (0,663 mMol) 4-(Dimethylamino)pyridin in 18 ml absolutem Methylenchlorid gelöst und dann zur gekühlten Lösung 0,992 g (4,801 mMol) Dicyclohexylcarbodiimid zugegeben. Nach kurzer Zeit beginnt Dicyclohexylharnstoff auszufallen. Nach 1 Stunde bei 2°C und 1,25 Stunden bei Raumtemperatur wird der Niederschlag abgenutscht und mit Methylenchlorid und Hexan nachgewaschen. Durch Abdampfen des Filtrates im Vakuum erhält man 1,831 g rohen 6-Butyl-trans-decalin-2-carbonsäure-trans-4-cyanocyclohexylester, der zur Reinigung an 50 g Kieselgel chromatographiert wird. Toluol und Toluol/0,5 bzw. 1% Aceton eluieren 1,240 g Substanz, die bis zum konstanten Schmelzpunkt und Klärpunkt aus Aceton/Hexan umkristallisiert und im Hochvakuum (0,01 mbar) bis zur Gewichtskonstanz getrocknet wird. Man erhält 6-Butyl-trans-decalin-2-carbonsäure-trans-4-cyano-1-cyclohexylester als farblose Kristalle; Smp. 103,3°C.

Beispiel 13

Zur Reinigung der gemäss Beispiel 5 erhaltenen rohen 6-Alkyl-trans-decalin-2-carbonsäuren kann man mit Vorteil auch die entsprechenden 6-Alkyl-trans-decalin-2-carbonsäureanilide herstellen:

3,144 g (12,5 mMol) rohe 6-Pentyl-trans-decalin-2-carbonsäure werden analog zu Beispiel 5 in das Säurechlorid übergeführt. Nach dem Entfernen des überschüssigen Thionylchlorids im Vakuum wird das rohe Säurechlorid in 15 ml absolutem Methylenchlorid gelöst und unter Rühren zu einer gekühlten Lösung von 2,56 g (27,5 mMol) Anilin in 35 ml absolutem Methylenchlorid getropft, wobei sich sofort ein Niederschlag von Anilin-hydrochlorid bildet. Nach 2,5 Stunden Rühren bei Raumtemperatur wird mit 50 ml Wasser versetzt

und erschöpfend mit Methylenchlorid extrahiert. Die organischen Extrakte werden mit Wasser gewaschen, über Natriumsulfat getrocknet und das Lösungsmittel im Vakuum entfernt. Der kristalline Rückstand von rohem 6-Pentyl-trans-decalin-2-carbonsäureanilid wird z.B. aus Aethanol bis zum konstanten Schmelzpunkt und Verschwinden der Verunreinigungen im Gaschromatogramm umkristallisiert. Man erhält so das reine farblose 6-Pentyl-trans-decalin-2-carbonsäureanilid; Smp. 177,9-178,6°C.

In analoger Weise können folgende Verbindungen hergestellt werden:

6-Methyl-trans-decalin-2-carbonsäureanilid;
Smp. 188,2-189,4°C
6-Aethyl-trans-decalin-2-carbonsäureanilid;
Smp. 189,4-190,8°C
6-Propyl-trans-decalin-2-carbonsäureanilid;
Smp. 194,5-196,3°C
6-Butyl-trans-decalin-2-carbonsäureanilid;
Smp. 185,7-186,7°C
6-Heptyl-trans-decalin-2-carbonsäureanilid;
Smp. 171,9-173,0°C.

Die Anilide können durch Hydrolyse, z.B. mit Kaliumhydroxid in Aethylenglykol bei Siedetemperatur und nachfolgendes Ansäuern wieder die reinen 6-Alkyl-trans-decalin-2-carbonsäuren übergeführt werden.

## Patentansprüche

1. Verbindungen der allgemeinen Formel

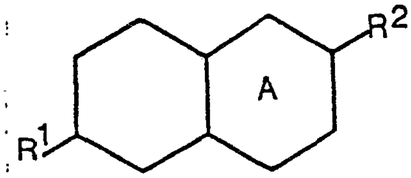

I

worin Ring A gesättigt oder aromatisch ist und ein gegebenenfalls vorhandener gesättigter Ring A mit dem zweiten Ring trans-verknüpft ist; $R^1$ eine geradkettige Alkyl- oder Alkoxygruppe mit 1 bis 11 Kohlenstoffatomen bezeichnet; $R^2$ Cyano, eine geradkettige Alkylgruppe mit 1 bis 11 Kohlenstoffatomen, eine Estergruppe der allgemeinen Formel

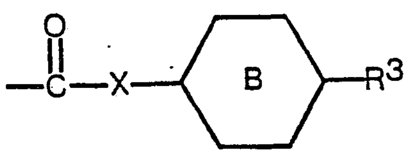

II

oder, sofern Ring A gesättigt ist, zusätzlich eine geradkettige Alkoxygruppe mit 1 bis 11 Kohlenstoffatomen bedeutet; in der Estergruppe der Formel II Ring B entweder aromatisch ist und X Sauerstoff oder Schwefel und $R^3$ Cyano oder eine geradkettige Alkyl- oder Alkoxygruppe mit 1 bis 10 Kohlenstoffatomen bezeichnet, oder Ring B einen trans-1,4-disubstituierten Cyclohexanring und X Sauerstoff und $R^3$ Cyano oder eine geradkettige Alkylgruppe mit 1 bis 10 Kohlenstoffatomen bedeutet; und die Gesamtzahl der Kohlenstoffatome in den Alkyl- und/oder Alkoxygruppen höchstens

12 beträgt.

2. Verbindungen nach Anspruch 1, dadurch gekennzeichnet, dass Ring A gesättigt ist.

3. Verbindungen nach Anspruch 1 oder 2, dadurch gekennzeichnet, dass der Rest $R^2$ eine Estergruppe der Formel II bedeutet.

4. Verbindungen nach Anspruch 3, dadurch gekennzeichnet, dass Ring B in der Estergruppe der Formel II aromatisch ist.

5. Verbindungen nach Anspruch 3 oder 4, dadurch gekennzeichnet, dass X in der Estergruppe der Formel II Sauerstoff bedeutet.

6. Verbindungen nach einem der Ansprüche 3 bis 5, dadurch gekennzeichnet, dass $R^3$ in der Estergruppe der Formel II Cyano oder eine geradkettige Alkylgruppe, vorzugsweise Cyano bedeutet.

7. Verbindungen nach Anspruch 1 oder 2, dadurch gekennzeichnet, dass $R^2$ Cyano, eine geradkettige Alkylgruppe oder, sofern Ring A gesättigt ist, zusätzlich eine geradkettige Alkoxygruppe bedeutet.

8. Verbindungen nach Anspruch 7, dadurch gekennzeichnet, dass $R^2$ Cyano oder eine geradkettige Alkylgruppe bedeutet.

9. Verbindungen nach einem der Ansprüche 1 bis 9, dadurch gekennzeichnet, dass $R^1$ eine geradkettige Alkylgruppe bedeutet.

10. Verbindungen der Formel I, gemäss Anspruch 1 als Komponenten in Flüssigkristallmischungen.

11. Flüssigkristallines Gemisch mit mindestens 2 Komponenten, dadurch gekennzeichnet, dass mindestens eine Komponente eine Verbindung der Formel I gemäss Anspruch 1 ist.

12. Verfahren zur Herstellung von Verbindungen der allgemeinen Formel

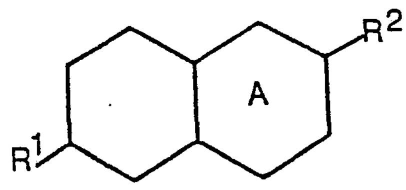

I

worin Ring A gesättigt oder aromatisch ist und ein gegebenenfalls vorhandener gesättigter Ring A mit dem zweiten Ring trans-verknüpft ist; $R^1$ eine geradkettige Alkyl- oder Alkoxygruppe mit 1 bis 11 Kohlenstoffatomen bezeichnet; $R^2$ Cyano, eine geradkettige Alkylgruppe mit 1 bis 11 Kohlenstoffatomen, eine Estergruppe der allgemeinen Formel

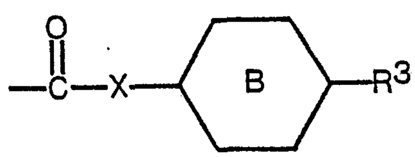

II

oder, sofern Ring A gesättigt ist, zusätzlich eine geradkettige Alkoxygruppe mit 1 bis 11 Kohlenstoffatomen bedeutet; in der Estergruppe der Formel II Ring B entweder aromatisch ist und X Sauerstoff oder Schwefel und $R^3$ Cyano oder eine geradkettige Alkyl- oder Alkoxygruppe mit 1 bis 10 Kohlenstoffatomen bezeichnet, oder Ring B einen trans-1,4-disubstituierten

Cyclohexanring und X Sauerstoff und $R^3$ Cyano oder eine geradkettige Alkylgruppe mit 1 bis 10 Kohlenstoffatomen bedeutet; und die Gesamtzahl der Kohlenstoffatome in den Alkyl- und/oder Alkoxygruppen höchstens 12 beträgt,

dadurch gekennzeichnet, dass man

a) zur Herstellung der Verbindungen der Formel I, worin $R^2$ eine Estergruppe der Formel II bedeutet, eine Verbindung der allgemeinen Formel

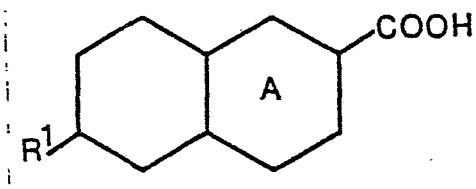

III

worin $R^1$ und A die obige Bedeutung haben, oder ein reaktionsfähiges Derivat hiervon mit einer Verbindung der allgemeinen Formel

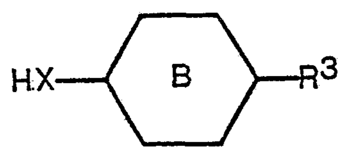

IV

worin X, B und $R^3$ die obige Bedeutung haben, oder einem geeigneten Salz hiervon verestert,

b) zur Herstellung der Verbindungen der Formel I, worin $R^2$ Cyano bedeutet, eine Verbindung der allgemeinen Formel

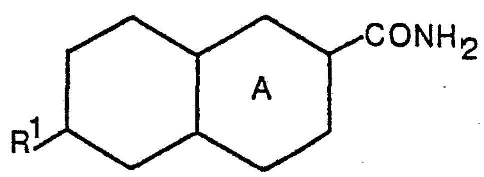

V

worin $R^1$ und A die obige Bedeutung haben, dehydratisiert,

c) zur Herstellung der Verbindungen der Formel I, worin $R^2$ eine geradkettige Alkylgruppe mit 1 bis 11 Kohlenstoffatomen bedeutet, eine Verbindung der allgemeinen Formel

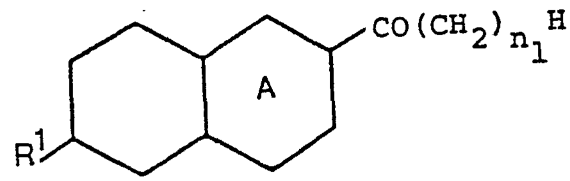

VI

worin $n_1$ eine ganze Zahl von 0 bis 10 bezeichnet und $R^1$ und A die obige Bedeutung haben,

mit Hydrazin in Gegenwart einer Base umsetzt,

d) zur Herstellung der Verbindungen der Formel I, worin $R^2$ eine geradkettige Alkylgruppe mit 2 bis 11 Kohlenstoffatomen bedeutet, eine Verbindung der allgemeinen Formel

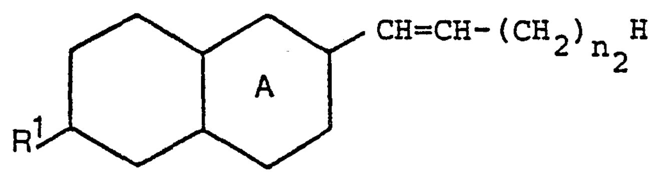

VII

worin $n_2$ eine ganze Zahl von 0 bis 9 bezeichnet und $R^1$ und A die obige Bedeutung haben,

katalytisch hydriert,

e) zur Herstellung der Verbindungen der Formel I, worin Ring A gesättigt ist und $R^2$ eine geradkettige Alkoxygruppe darstellt, eine Verbindung der allgemeinen Formel

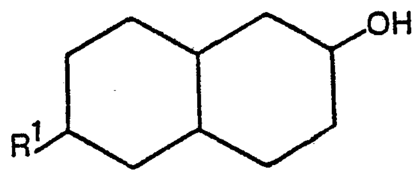

VIII

worin $R^1$ die obige Bedeutung hat, veräthert.

13. Verwendung der Verbindungen der Formel I gemäss Anspruch 1 für elektro-optische Zwecke.

14. Verbindungen der allgemeinen Formel

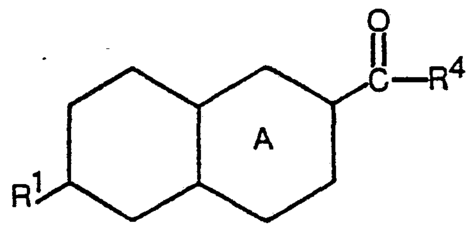

X

worin Ring A gesättigt oder aromatisch ist und ein gegebenenfalls vorhandener gesättigter Ring A mit dem zweiten Ring trans-verknüpft ist, $R^1$ eine geradkettige Alkyl- oder Alkoxygruppe mit 1 bis 11 Kohlenstoffatomen bedeutet und $R^4$ Wasserstoff, Chlor, die Hydroxy- oder Aminogruppe oder eine geradkettige Alkylgruppe mit 1 bis 10 Kohlenstoffatomen darstellt.

***